(19) Europäisches Patentamt
European Patent Office
Office européen des brevets

(11) EP 0 455 271 B1

(12) **EUROPEAN PATENT SPECIFICATION**

(45) Date of publication and mention of the grant of the patent:
**20.08.1997 Bulletin 1997/34**

(51) Int. Cl.[6]: **A61K 31/00**

(21) Application number: **91111471.8**

(22) Date of filing: **10.06.1987**

(54) **Anti-inflammatory compositions**

Entzündungshemmende Mittel

Compositions anti-inflammatoires

(84) Designated Contracting States:
**AT BE CH DE FR GB IT LI LU NL SE**

(30) Priority: **11.06.1986 US 872812**

(43) Date of publication of application:
**06.11.1991 Bulletin 1991/45**

(62) Document number(s) of the earlier application(s) in accordance with Art. 76 EPC:
**87904184.6 / 0 310 622**

(73) Proprietor: **PROCYON PHARMACEUTICALS, INC.**
**Woburn, Massachusetts 01801 (US)**

(72) Inventor: **Driedger, Paul E.**
**Winchester, Massachusetts 01890 (US)**

(74) Representative: **Holdcroft, James Gerald, Dr. et al**
**Graham Watt & Co.,**
**Riverhead**
**Sevenoaks, Kent TN13 2BN (GB)**

(56) References cited:
**US-A- 4 560 774**

- **CHEMICAL ABSTRACTS, vol. 70, no. 11, 17th March 1969, page 359, abstract no. 47622q, Columbus, Ohio, US; H. U. SCHAIRER et al.: "Chemistry of phorbol. IV. Polybenzoates and polyacetates of phorbol and 3-phorbolol and functional derivates of the allyl group of phorbol"**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 455 271 B1

Printed by Rank Xerox (UK) Business Services
2.14.12/3.4

## Description

Protein kinase C is an enzyme found in nearly all animal tissues and animal cells that have been examined. Its identity is generally established by its ability to phosphorylate proteins when adenosine triphosphate, calcium ions and phospholipid cofactors are present, with greatly reduced activity when these cofactors are absent. Protein kinase C activity is substantially stimulated by certain 1,2-sn-diacylglycerols that bind specifically and stoichiometrically to a recognition site on the enzyme. Stimulation of protein kinase C by these diacylglycerols has been shown to be an important physiological event that mediates the actions of a wide variety of hormones, neurotransmitters, and other biological control factors such as histamine, vasopressin, alpha-adrenergic agonists, dopamine agonists, muscarinic cholinergic agonists, platelet activating factor, etc. [see Y. Nishizuka, Nature 308 693-698 (1984) and Science 225 1365-1370 (1984) for reviews].

The biological role of protein kinase C is also of great interest because of the discovery that certain very powerful tumor promoting substances activate this enzyme by binding specifically and with very high affinity to the diacylglycerol binding site on the enzyme. In addition to diacylglycerols, there are at present five other known classes of compounds that bind to this site, including diterpenes such as the phorbol esters, indole alkaloids (indolactams) such as the teleocidins, lyngbyatoxin, and indolactam V, polyacetates such as the aplysiatoxins and oscillatoxins, certain derivatives of diaminobenzyl alcohol, and the macrocyclic lactones of the bryostatin class. The phorbol esters have long been known as powerful tumor promoters, the teleocidins and aplysiatoxins are now known to have this activity, and it appears likely that additional classes of compounds will be found to have the toxic and tumor promoting activities associated with the capability to bind to the diacylglycerol site of protein kinase C and thus activate the enzyme. Other toxicities of these agents when administered to animals include lung injury and profound changes in blood elements, such as leukopenia and neuropenia.

In addition to potent tumor promoting activity, these six classes of compounds, collectively referred to as the "phorboids", display a vast range of biological activities, as would be expected from the widespread distribution of their target enzyme. Some of these activities, like tumor promotion, indicate the involvement of protein kinase C in important normal or pathological processes in animals. Thus, the phorboids are potent skin inflammatory agents, cause smooth muscle contraction in several tissues, alter immune system function and can be used to cause a variety of other normal or pathological responses. Related disease states such as the development of cancer, the onset and/or maintenance of inflammatory disease, the role of vaso-constriction in hypertension, the role of broncho-constriction in asthma, the role of cholinergic, adrenergic, and dopaminergic synapses in diseases of the central/peripheral nervous systems, may be mediated in vivo by the stimulation of protein kinase C by diacylglycerols, the latter being generated in the cell by pathological agents or conditions.

In analyzing the activity of a pharmaceutical or other bioactive compound, it is useful to consider two properties: the efficacy, defined as the capability to elicit a full or partial biological result, such as complete displacement of a ligand from its receptor site or the complete inhibition of inflammation or edema caused by a standard stimulus; and the potency, defined as that amount or concentration of drug that causes 50% of the full response (often abbreviated as the $ED_{50}$). It is frequently the case within a given class of pharmaceutical agents that individual members of the class all have equal efficacy, i.e. they each can generate a full biological effect, but they show differing potencies. Thus, the structural modifications within such a class affect only the amount necessary to achieve a given result, and the modified compounds otherwise have generally the same central biological characteristic. There may also be differences between members of such a class as regards properties other than the central biological characteristic; for example, members of the class might differ in side effects or toxicity.

Well-known pharmaceuticals that have been in extensive use for years or decades show a wide range of optimal therapueutic potencies. Aspirin, for example, is often taken in multi-gram amounts per day for treatment of inflammation or arthritis, and detailed analyses of its mechanism of action in vitro show that a concentration in the millimolar range is required. In contrast, steroid-based topical anti-inflammatory compounds such as fluocinolone acetonide are many thousand-fold more potent, and, beyond this, some oral contraceptive agents are prescribed in daily doses in the microgram range. Thus, although high potency is generally advantageous for a pharmaceutical, it is not an absolute requirement.

Several thousand of the high skin-inflammatory and tumor-promoting phorboids have been reported in the literature, including numerous examples on which minor chemical modifications have been made [see Evans and Soper, Lloydia 41 193-233 (1978) and references cited therein]. The structures of these phorboids can be compared, and their activities for inflammation and tumor promotion can be analyzed from the perspective of efficacy and potency. The structures of the different classes of phorboids vary quite markedly from one to the other class for structural comparisons), yet widespread testing of their biological activities has shown that these classes have generally very similar biological properties. In particular, the thousands of known phorboids of the highly potent diterpene, indolactam, and polyacetate classes appear to have, with very minor exceptions, virtually identical efficacies as skin irritants and tumor promoters [T. Sugimura, Gann 73 499-507 (1982)]. The exceptions involve a few compounds that have a short duration of irritant activity and/or manifest diminished tumor promoting activity, perhaps due to toxicity or secondary parameters

such as differing metabolic destruction rates.

In contrast to the essentially equal efficacies among the vast majority of phorboids, their relative potencies cover a wide range, as measured in inflammation and promotion tests and as measured in numerous other in vivo and in vitro systems. Example compounds can be found in the diterpene, indolactam, and polyacetate classes that have nearly equal, very high potencies. At the same time there are compounds in each of these classes which embody significant structural changes that do not diminish efficacy but do result in potency decreases of 10-fold to 100,000-fold or more [see, for example, Driedger and Blumberg, Cancer Res. 37 3257-3265 (1977), Cancer Res. 39 714-719 (1979)]. Thus, all these compounds appear to be capable of achieving generally the same biological results, and merely differ in the amount which must be used to obtain a given result.

In vitro measurements of biochemical properties provide an even more sensitive method for comparing the properties of the various phorboids. For example, using a radioactively labeled phorboid such as [$^{3}$H]phorbol 12,13-dibutyrate or [$^{3}$H]lyngbyatoxin, one can measure the potency of a test compound as a competitive ligand for the diacylgylcerol binding site on protein kinase C. Alternatively, one can measure the ability of a given phorboid to stimulate the protein kinase C-mediated incorporation of radioactive phosphate from [$^{32}$P]adenosine triphosphate into a standard acceptor substrate such as histone HI. Tests of this nature reveal a difference in potency between given phorboid agonists of as much as 10,000,000-fold or more [Dunn and Blumberg, Cancer Res. 43 4632-4637 (1983), Table 1].

These basic data regarding the phorboid agonists are an important consideration because they underscore the concept that the structural differences among these previously known phorboids, especially the diterpenes, indolactams, polyacetates, and bryostatins, generally do rot affect their efficacies as toxic agonists, and indeed a wide variety of structural changes are tolerated in this regard. Such changes generally alter potency only and do not provide agents with therapeutic utility, since they retain their toxicity.

Some minor changes in phorboid structure are known to result in inactive compounds, such as a stereochemical change from 4-beta to 4-alpha in the phorbol series, and indeed some of the diterpene skeleton structures carry hydroxy groups that must be esterified in order for inflammatory activity to be observed. However, these inactive compounds are quite few in number among the known phorboids, and no therapeutic utility has been demonstrated for them.

The phorbol esters, indolactams, polyacetates, diaminobenzyl alcohols, and bryostatins are generally found in plants, molds, and algae, or are synthetic in origin. Although they are found in many parts of the world, normal human contact with them is thought to be low. In contrast, the diacylglycerols are part of the functioning of virtually every type of animal cell except erythrocytes of some species, and thus the undesirable activation of protein kinase C by the diacylglycerols may have a very widespread role in human diseases.

Thus, new compounds, capable of blocking the activation of protein kinase C by acting as specific pharmacological antagonists of the diacylglycerols, would be valuable agents in the prevention and treatment of a wide variety of diseases in animals and humans.

There may be several different forms of protein kinase C each having different biological roles. The stimulation of one form can lead to undesirable results such as inflammation or the development of cancer, while stimulation of another form of the enzyme might produce beneficial effects, such as the abrogation of inflammation or the secretion of useful bioregulatory factors such as hormones and interferons.

Moreover, the exact correspondence between diacylglycerol binding sites and protein kinase C has not been fully explored, and there may be several different such binding sites with differential affinities for diacylglycerols and other phorboids. Indeed there is published evidence for several distinct classes of phorboid binding sites in various tissues and cell types (Dunn and Blumberg, op. cit.). However, in this study, even the ligands showing the clearest differences in affinity for these distinct classes are only selective by a factor of 10-100 dissociation constant. Thus, other new compounds, capable of selectively activating one useful, but not another, deleterious, diacylglycerol target site, would also be valuable agents for the prevention or treatment of inflammation.

Earlier efforts to use the previously known phorboids themselves or to modify the structures of the known phorboids, have not been successful in producing useful compounds.

It has been known for some time that several of the toxic, inflammatory and tumor-promoting compounds such as phorbol 12-tigliate 13-decanoate, mezerein, lyngbyatoxin and aplysiatoxin have anti-leukemic activity in mouse model tests. However, these compounds are all extremely toxic and are cancer suspect agents, thus eliminating them from consideration as human therapeutic agents.

Ganong et al. [Proc. Nat. Acad. Sci. 83 1184-1188 (1986)] tested a series of diacylglycerols and found no antagonistic activity in that series against the standard agonist 1,2-dioctanoylglycerol. It is of particular note that several compounds tested in this work were modified in the hydroxymethyl portion of the diacylglycerol molecule, and these modifications produced only a loss of activity or a weakened activity that was not distinguishable from the agonist activity of 1,2-dioctanoylglycerol itself. These hydroxymethyl-modified compounds were not antagonists in these tests and no utility was found. Similarly, Thielmann and Hecker [Forsch. Krebsforsch. Vol. VII, pp. 171-179 (1969), New York: Schattauer] found only a complete loss of biological activity in their study when the hydroxy group of the hydroxymethyl on phorbol 12,13-didecanoate was replaced with hydrogen or chlorine. Schmidt and Hecker [H. Lettre and G. Wagner

(eds.), Aktuelle Probleme aus dem Gebiet der Cancerologie, Vol. III, 3rd Heidelberg Symposium, pp. 98-108. Berlin: Springer Verlag, 1971] also found that oxidation of the hydroxymethyl of phorbol 12,13-didecanoate to a carboxylic acid caused complete loss of activity in the assays used.

The hydroxymethyl group of the known phorboids has been thought to be required for biological activity, as detailed by Hecker (Carcinogenesis, Vol. 2, eds. Slaga, Sivak and Boutwell, Raven Press, New York, 1978, pp. 11-48 and references cited therein). Indeed, it is stated therein that the replacement of the 20-hydroxyl in a phorbol ester "results in complete loss of biological activity". In another study, replacement of the hydroxy group of the hydroxymethyl (located at carbon 14) by chlorine or hydrogen in indolactam V gave rise to compounds with agonist activity weaker than but otherwise not distinguished from the agonist activity of the very toxic teleocidin class of tumor promoters [Irie et al., Int. J. Cancer 36 485-488 (1985)]. Thus no utility was found.

Schmidt and Hecker (Carcinogenesis, Vol. 7, ed. by E. Hecker et al., Raven Press, New York, 1982, pp. 57-63) studied the abilities of a series of diterpene phorboids to inhibit tumor promotion by the standard phorboid agonist tumor promoter phorbol 12-myristate 13-acetate (PMA). They found that, at low doses, some short-chain ester derivatives of phorbol were able to block the tumor promotion by PMA. However, all of the compounds that were active as antagonists at low doses are also very efficacious skin irritants themselves at higher doses and most of them are also known to have tumor promoting activity. Thus, these short-chain esters still have toxic inflammatory and tumor promoting activity and thus have no therapeutic value. In this publication it was also noted that a phorbol 12-ester, namely phorbol 12-myristate, was without activity as an antagonist of PMA-induced tumor promotion. Theilmann and Hecker (op. cit.) also found phorbol 12-decanoate and phorbol 12-myristate to be inactive in the functional tests used, and stated that the 13-OH must be esterified to obtain active compounds.

The scope of the invention is defined in the appended claims.

This invention pertains to phorboid derivatives which block the toxic effects of the hydroxymethyl-containing phorboids and lack the toxic properties of previously available phorboids. These phorboid derivatives of very diverse structures have utility as anti-inflammatory agents, anti-cancer and anti-leukemic agents, anti-asthmatic and anti-hypertensive agents, as modulators of in vivo immune function, as stimulators of the production of lymphokines such as interferon and the interleukins, as stimulants of hair growth on bald or partially bald scalp, as central nervous system pharmaceuticals for several pathological conditions, and as xenobiotics for achieving the control of parasites. The structural features associated with the non-toxicity and protein kinase C antagonism of these compounds relate primarily to the hydroxymethyl or 1-hydroxyethyl group found in each of the toxic parents. Specific modification of the latter chemical groupings yields non-toxic anti-inflammatory compounds, whereas any of a very wide variety of changes in other parts of the parent structures, including but not limited to diterpenes, indole alkaloids, polyacetates, diaminobenzyl alcohol derivatives, aplysiatoxins, and bryostatinoids, have very markedly less effect on the overall biological properties of the derivatives, other than changes in potency. This invention also provides new compounds that discriminate between phorboid receptor subtypes, with relative binding activities differing by 10,000-fold or more.

A comparison of the structures of the previously known phorboids reveals the features that are critical for this invention, as shown below.

TYPICAL DITERPENE-TYPE PHORBOID AGONISTS

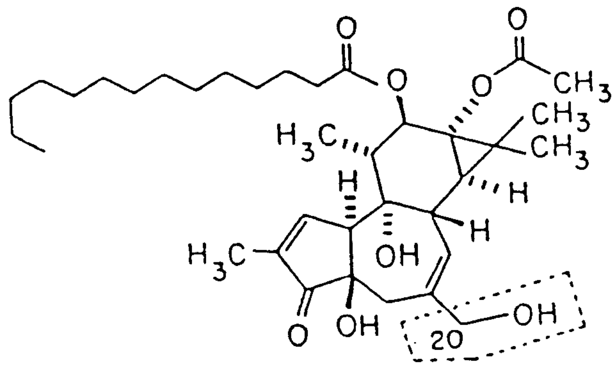

Phorbol 12-Myristate 13-Acetate (PMA)

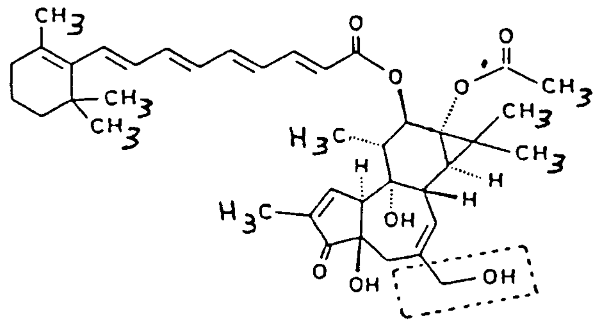

Phorbol 12-Retinoate 13-Acetate

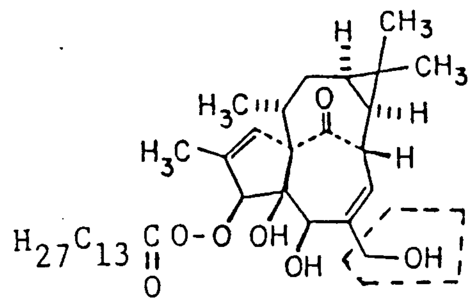

Ingenol 3-Tetradecanoate

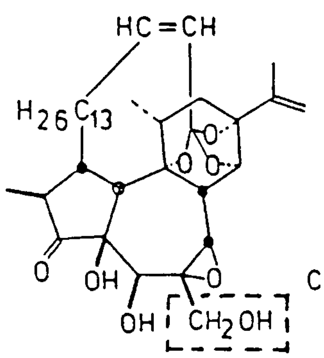

Synaptolepsis Factor $K_1$

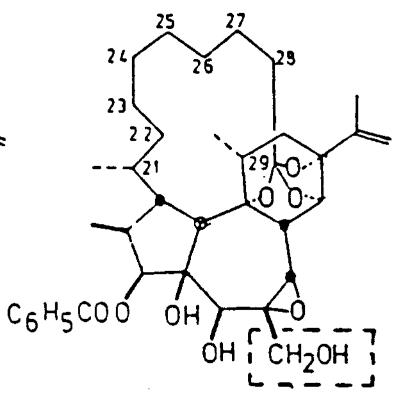

Pimelea Factor $P_2$

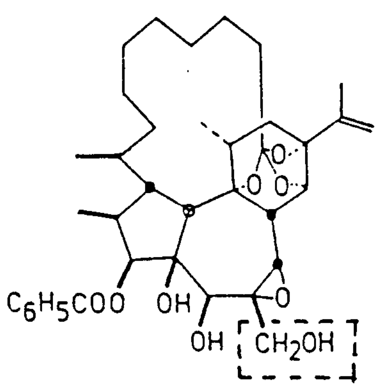

Daphnopsis Factor $R_6$

TYPICAL DITERPENE-TYPE PHORBOID AGONISTS (cont.)

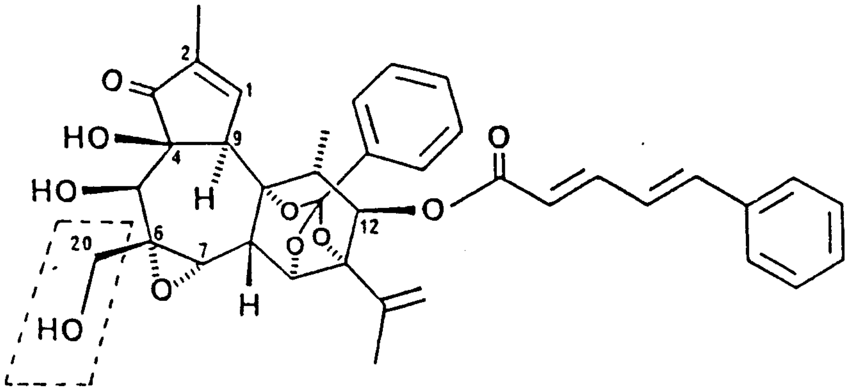

MEZEREIN

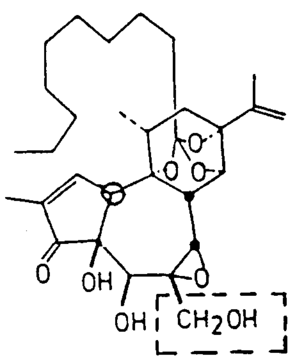

Simplexin

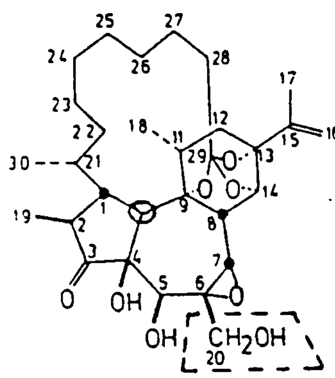

Pimelea Factor $S_7$

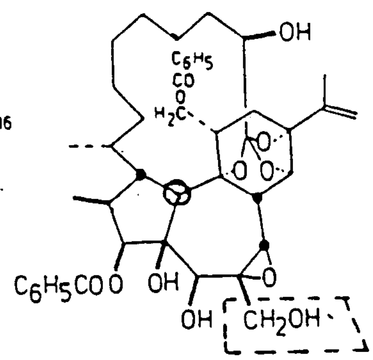

Gnidimacrin

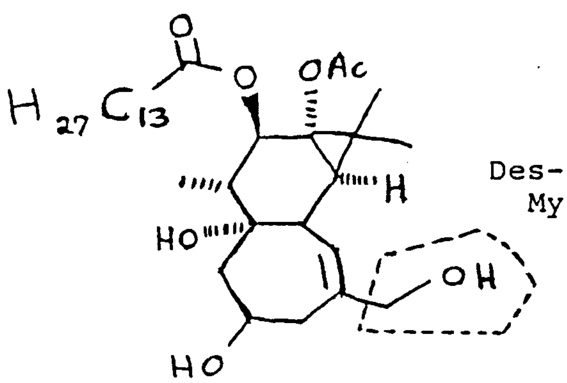

Des-(Ring A)-phorbol 12-Myristate 13-Acetate

TYPICAL INDOLACTAM-TYPE PHORBOID AGONISTS

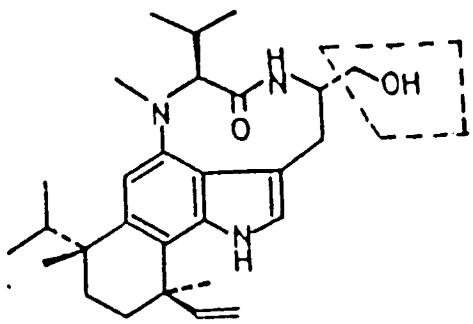

Teleocidin $B_4$

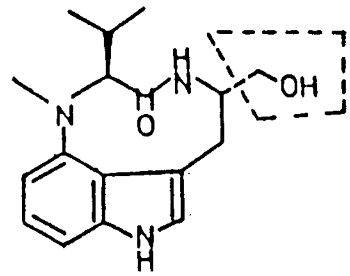

Indolactam V

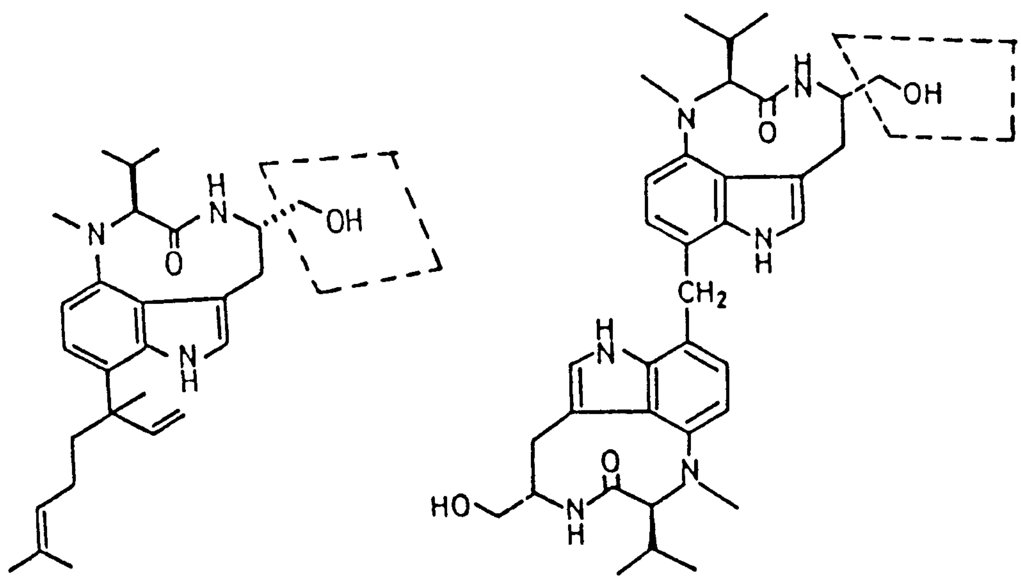

Lyngbyatoxin A

Blastmycetin A

Typical Diaminobenzyl Alcohol-type Phorboid Agonists

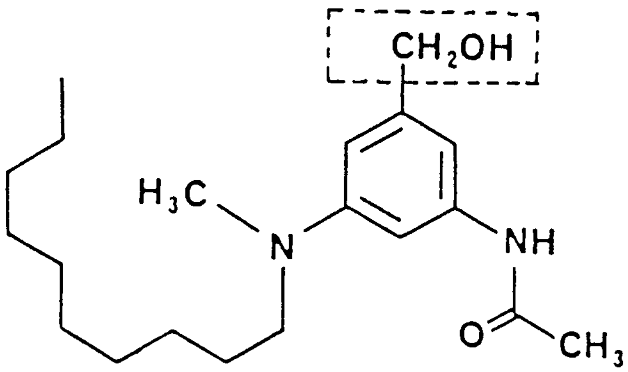

3-Acetylamino-5-(N-decyl-N-methylamino)benzyl Alcohol

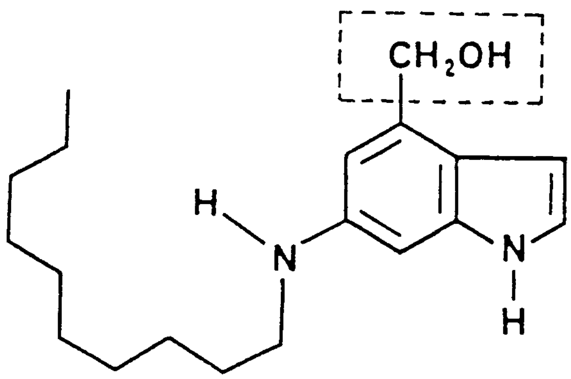

6-(N-decylamino)-4-hydroxymethylindole

Typical Diacylglycerol-type Phorboid Agonists

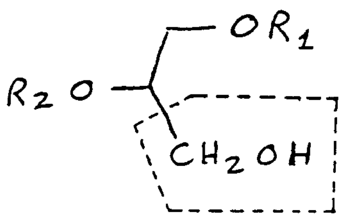

$R_1$ = OLEOYL, $R_2$ = ACETYL

$R_1$ = STEAROYL, $R_2$ = ARACHIDONOYL

$R_1$ = $R_2$ = OCTANOYL

---

Typical Polyacetate-type Phorboid Agonists

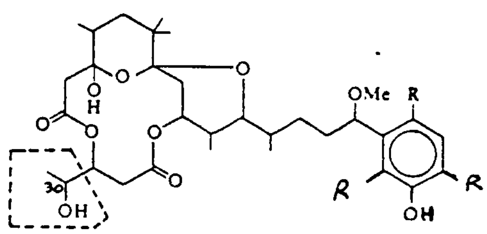

R's = H or Br

(Aplysiatoxin and its debromo, bromo, and dibromo derivatives)

TYPICAL BRYOSTATIN-TYPE PHORBOID AGONISTS

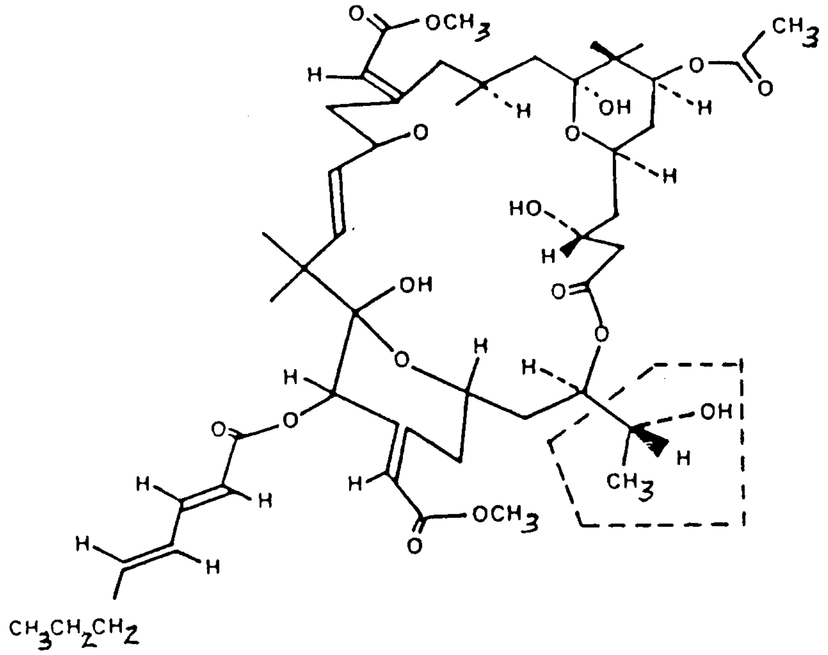

Bryostatin 1

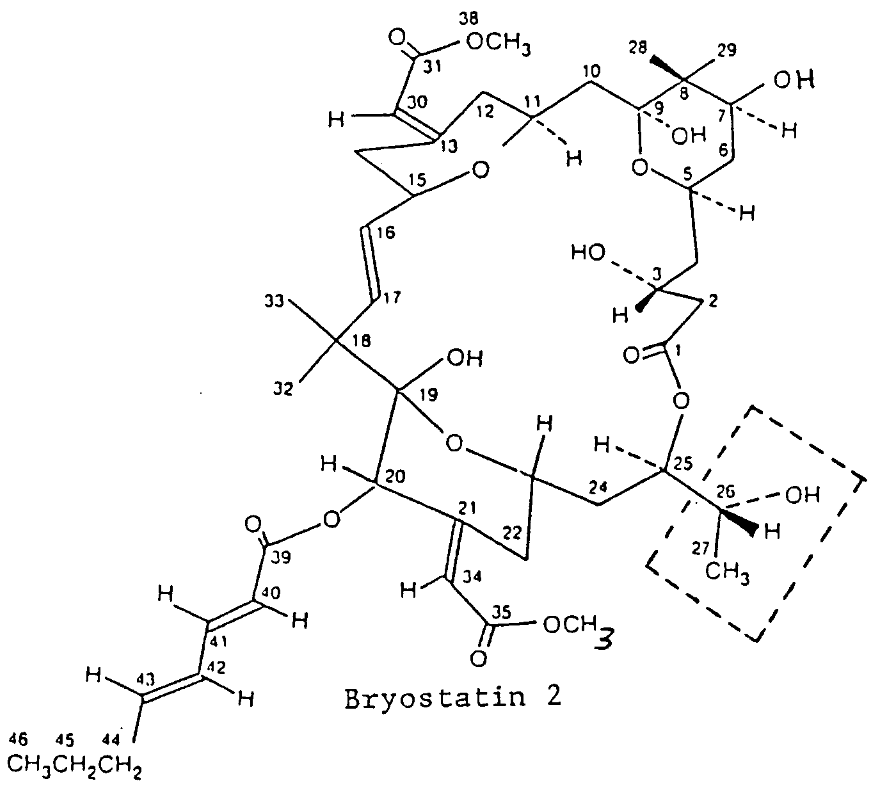

Bryostatin 2

It can be seen that the phorboids depicted have exceeding diverse structural elements, with one prominent exception, namely that each contains a hydroxymethyl or 1-hydroxyethyl group (indicated by the dashed-line boxes in each structure). In each case the phorboid depicted is among the most potent of its particular structural class, and among the classes the diterpenes, indolactams and polyacetates have members of especially high potency.

The hydroxymethyl and 1-hydroxyethyl feature common to all the classes of phorboids is the primary focus of this invention. Generally the phorboid derivatives can be represented by the formula:

$$P - G$$

wherein P represents a radical, formally derived from a parent compound, which compound:

a. binds reversibly or irreversibly to a diacylglycerol-type receptor; and/or
b. activates any form of the enzyme protein kinase C; and
c. contains an hydroxymethyl or 1-hydroxyethyl group bonded to a carbon atom; and

wherein G is any group either i) singly or doubly bonded to the carbon atom of the parent compound in place of the hydroxymethyl or 1-hydroxyethyl group; or ii) singly or doubly bonded to a carbon atom immediately adjacent to the carbon atom to which the hydroxymethyl or 1-hydroxyethyl group is bound in the parent compound; and wherein the hydroxymethyl or 1-hydroxyethyl group of the parent compound is absent or has been replaced by G.

More specifically, they are represented by the formula:

$$P_o - S_o\text{-}E_o$$

which depicts a phorboid "parent" compound radical $P_o$ modified by a moiety $S_oE_o$.

In general, a parent compound is a compound which:

a. binds reversibly or irreversibly to a diacylglycerol-type receptor; and/or
b. activates any form of the enzyme protein kinase C; and
c. contains an hydroxymethyl or 1-hydroxyethyl group bonded to a carbon atom.

For example, a parent compound can be a diterpenoid activator of protein kinase C; an aromatic heterocyclic activator of protein kinase of the indole, indene, benzofuran, or benzothiophene class; a polyacetate-derived activator of protein kinase C; an activator of protein kinase C of the diacylglycerol or diacyloxybutanol class; an activator of protein kinase C of the diaminobenzyl alcohol class; or a protein kinase C activator of the bryostatin class.

$S_o$-$E_o$ represents a modifying moiety which is either:

i) singly or doubly bonded to the carbon atom of the parent compound $P_o$ in place of the hydroxymethyl or 1-hydroxyethyl group; or
ii) singly or doubly bonded to a carbon immediately adjacent to the carbon atom to which the hydroxymethyl or 1-hydroxyethyl is bonded.

In the $S_o$-$E_o$ moiety, $S_o$ can be a saturated or unsaturated, straight or branched chain of atoms which separates $P_o$ and $E_o$ by a linear count of at least two but not more than 6 atoms and contains one to six heteroatoms selected from oxygen, nitrogen and sulfur, a phosphorus atom, or up to 12 halogen atoms; provided that the total number of atoms does not exceed about 35, and in some cases $S_o$ may be a single or double bond; and $E_o$ can be a saturated or singly or multiply unsaturated group containing 1 to 8 carbon atoms and optionally containing 1 to 12 halogen atoms, 1 to 4 heteroatoms selected independently from oxygen, nitrogen and sulfur and one phosphorus atom. $S_oE_o$ taken together may also be a hydrogen, halogen, or ketonic oxygen atom or a hydroxy, amino, or thiol group singly or doubly bonded to the carbon atom of the parent compound $P_o$ in place of the hydroxymethyl or 1-hydroxyethyl group.

## Detailed Description of the Invention

All six classes of the phorboids have one structural element in common. Each prototypical member of these classes has either a hydroxymethyl group or a 1-hydroxyethyl group. The design of the phorboid derivatives of this invention is based on the finding that the hydroxymethyl and 1-hydroxyethyl groups which previously were thought to be required for biological activity of phorboids containing these groups can be replaced by other substituents of very diverse nature, and the resulting compounds both block the toxic effects of the hydroxymethyl-containing phorboids and lack the toxic properties associated with the previously available phorboids. These new compounds thus have utility as anti-inflammatory agents, for example. Although the replacement of the hydroxymethyl group or 1-hydroxyethyl group is very specific and leads to an extreme and profound change in biological properties; a very wide range of structural alterations in the remainder of the novel compounds can be tolerated without material loss of their anti-inflammatory activity.

The phorboid derivatives are generally represented by the formula:

P - G

The formula depicts a radical, P, derived from a parent compound, which compound:

a. binds reversibly or irreversibly to a diacylglycerol-type receptor; and/or
b. activates any form of the enzyme protein kinase C; and
c. contains an hydroxymethyl or 1-hydroxyethyl group bonded to a carbon atom; and

wherein G is any group either i) singly or doubly bonded to the carbon atom of the parent compound in place of the hydroxymethyl of 1-hydroxyethyl group; or ii) singly or doubly bonded to a carbon atom immediately adjacent to the carbon atom to which the hydroxymethyl or 1-hydroxyethyl group is bound in the parent compound; and wherein the hydroxymethyl or 1-hydroxyethyl group of the parent compound is absent or has been replaced by G.

More specifically, they are represented by the formula:

$$P_o - S_o - E_o$$

The formula depicts a radical $P_o$, formally derived from a parent hydroxymethyl-containing phorboid compound, bonded to an $S_o$-$E_o$ moiety.

$P_o$ represents a radical formally derived from a compound which contains an hydroxymethyl (or the equivalent 1-hydroxyethyl) group and which binds reversibly or irreversibly to a diacylglycerol-type receptor and/or activates any form of the enzyme protein kinase C. $P_o$ may be formally derived from phorboids from any of the six classes listed below:

i) a diterpenoid activator of protein kinase C;
ii) an aromatic heterocyclic activator of protein kinase of the indole, indene, benzofuran, or benzothiophene class;
iii) a polyacetate-derived activator of protein kinase C;
iv) an activator of protein kinase C of the diacylglycerol or diacyloxybutanol class;
v) an activator of protein kinase C of the diaminobenzyl alcohol class; and
vi) a protein kinase C activator of the bryostatin class.

All of these phorboids contain an hydroxymethyl or 1-hydroxyethyl group which is known to be required for their toxic biological activity, such as inflammatory activity measured on the mouse ear.

$S_o$-$E_o$ represents a moiety which is either:

i) singly or doubly bonded to the carbon atom of the parent compound in place of the hydroxymethyl or 1-hydroxyethyl group; or
ii) singly or doubly bonded to a carbon immediately adjacent to the carbon atom to which the hydroxymethyl or 1-hydroxyethyl group is bound in the parent compound.

In this $S_o$-$E_o$ moiety, $S_o$ can be a saturated or unsaturated, straight or branched chain of atoms which separates $P_o$ and $E_o$ by a linear count of at least two but not more than 6 atoms and contains one to six heteroatoms selected from oxygen, nitrogen and sulfur, a phosphorus atom, or up to 12 halogen atoms; provided that the total number of atoms does not exceed about 35, and in some cases $S_o$ may be a single or double bond; and $E_o$ can be a saturated or singly or multiply unsaturated group containing 1 to 8 carbon atoms and optionally containing 1 to 12 halogen atoms, 1 to 4 heteroatoms selected independently from oxygen, nitrogen and sulfur and one phosphorus atom. $S_oE_o$ taken together may also be a hydrogen, halogen, or ketonic oxygen atom or a hydroxy, amino, or thiol group singly or doubly bonded to the carbon atom of the parent compound $P_o$ in place of the hydroxymethyl or 1-hydroxyethyl group.

More generally, the phorboid derivatives are represented as follows:

$$P_x - S_x - E_1$$

wherein $P_x$ can be selected from six different classes of compounds designated $P_1$-$P_6$ and defined below, wherein $S_x$ is selected from six different structural types as defined below and $E_1$ is as defined below.

$P_1$, $P_2$, $P_3$, $P_4$, $P_5$, and $P_6$ represent compounds of each of the six classes of known phorboids and are defined by the formulae below.

$P_1$ is a radical of the formula:

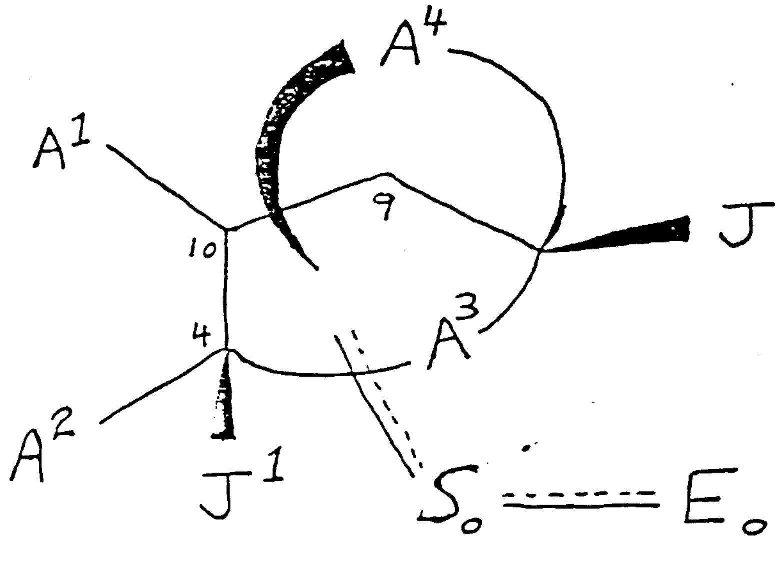

$(P_1)$

in the form of an individual isomer, an isomer mixture, a racemate or optical antipode, or a pharmaceutically acceptable salt thereof;

wherein $A^1$ and $A^2$ may be individually selected from hydrogen and a straight chain or branched chain, cyclic or acyclic, saturated, unsaturated and/or aromatic carbon- and/or heteroatom-containing substituent having not more than 34 carbon atoms, not more than 24 halogen atoms and not more than 6 heteroatoms selected from oxygen, nitrogen and sulfur; $A^1$ and $A^2$ taken together complete a 5- or 6-membered carbocyclic or heterocyclic ring, optionally substituted by one or more straight chain or branched chain, cyclic or acyclic, saturated, unsaturated, and/or aromatic carbon- and/or heteroatom-containing groups, which groups taken together contain a total of not more than 30 carbon atoms, not more than 24 halogen atoms and not more than 6 heteroatoms selected from oxygen, nitrogen, and sulfur; $A^3$ is a three atom chain which completes a 7-membered carbocyclic ring optionally substituted by one or more straight chain or branched chain, cyclic or acyclic, saturated, unsaturated, and/or aromatic carbon- and/or heteroatom-containing groups, which groups taken together, excluding $S_oE_o$, contain not more than 7 carbon atoms, not more than 4 halogen atoms, and not more than 5 heteroatoms selected from oxygen, nitrogen and sulfur; provided that, including $S_oE_o$, the middle carbon atom of $A^3$ is not substituted by hydroxymethyl, hydroxyethyl or 2-hydroxy-2-propyl;

$A^4$ completes a 6- or 7-membered carbocyclic or heterocyclic ring (connected in the beta configuration to either carbon atom 9 or 10), optionally substituted by one or more straight chain or branched chain cyclic or acyclic, saturated, unsaturated and/or aromatic carbon and/or heteroatom-containing groups, the group or groups optionally completing one or two additional rings by themselves and/or one to five additional rings when taken together with $A^1$, $A^2$, a ring formed by $A^1$ and $A^2$ together, and/or a bond to carbon atom 9, which groups taken together, include not more than 40 carbon atoms, not more than 24 halogen atoms, and not more than 10 heteroatoms selected from oxygen, nitrogen, and sulfur; $J^1$ is selected from hydrogen, fluoro, chloro, hydroxy, amino, mono- or di-lower-alkylamino, methyl, ethyl, vinyl, ethynyl, propargyl, cyano, methoxy, ethoxy, trifluoromethyl, 2-hydroxyethyl, 2-hydroxypropyl, 3-hydroxypropyl, acetoxy, propanoyloxy, acetyl, propanoyl, hydroxyacetyl, 2-hydroxypropanoyloxy, 3-hydroxypropanoyl, acetamido, propanamido, hydroxyacetamido, 2-hydroxypropanamido, or 3-hydroxypropanamido, (each of which must be situated in the beta position), or $J^1$ taken together with $A^1$, $A^2$, or a ring formed by $A^1$ together with $A^2$ completes a 3- to7-membered substituted or unsubstituted carbocyclic or heterocyclic ring; $J^2$ is selected from hydrogen, methyl, ethyl, hydroxymethyl, hydroxyethyl, vinyl, ethynyl, allyl, propargyl, n-propyl and iso-propyl; provided that if $P_1$ is a phorbol-12,13-dialkanoyl moiety then $S_oE_o$ may not be carboxy, carboxaldehyde, carboxaldehyde-2,4-dinitrophenylhydrazone, or alkanoyloxymethylene, and provided that if $P_1$ is a phorbol 12,13-di-0-acetyl moiety then $S_oE_o$ may not be cyano or aldoxime.

$P_2$ is a radical of the formula:

($P_2$)

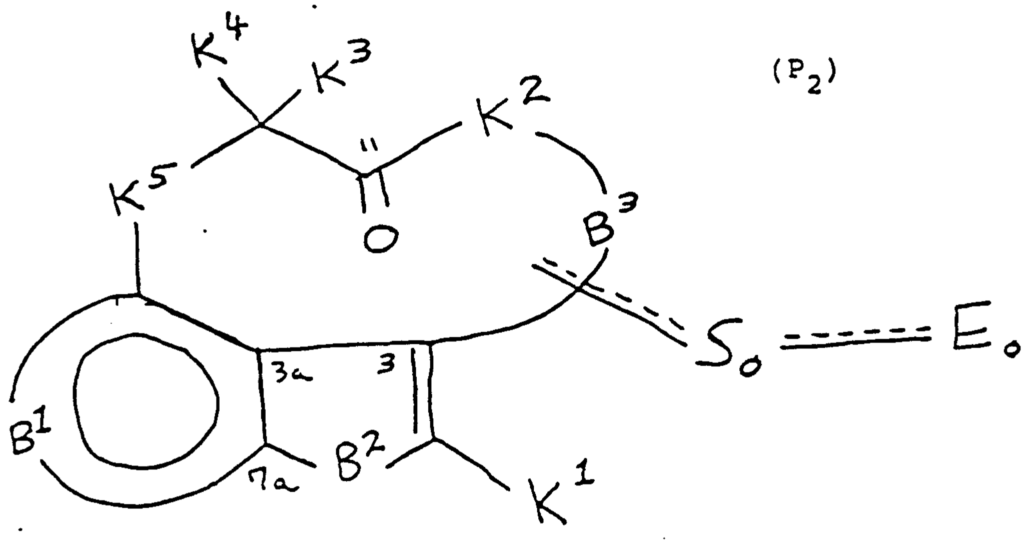

in the form of an individual isomer, an isomer mixture, a racemate or optical antipode, or a pharmaceutically acceptable salt thereof,

wherein $B^1$ completes a 6-membered carbocyclic or heterocyclic aromatic ring, optionally substituted by a straight chain or branched chain, cyclic or acyclic, saturated, unsaturated, and/or aromatic carbon- and/or heteroatom-containing groups which, taken together, contain not more than 40 carbon atoms, not more than 24 halogen atoms, and not more than 9 heteroatoms selected from oxygen, nitrogen, and sulfur, the groups being optionally connected to form 1-3 additional rings; $B^2$ is selected from oxygen, sulfur, sulfoxide, sulfone, and a carbon or nitrogen atom optionally substituted by a straight chain or branched chain, cyclic or acyclic, saturated, unsaturated, and/or aromatic carbon- and/or heteroatom-containing groups having not more than 15 carbon atoms, not more than 24 halogen atoms, and not more than 6 heteroatoms selected from oxygen, nitrogen, and sulfur, and $B^2$ may be linked to $B^1$ to form an additional carboxyclic or heterocyclic ring; $B^3$ is a 2-carbon chain optionally substituted by one or more straight chain or branched chain, cyclic or acyclic, saturated, unsaturated, and/or carbon- and/or heteroatom-containing groups, which groups taken together but excluding $S_oE_o$, contain not more than 8 carbon atoms, not more than 6 halogen atoms, and not more than 6 heteroatoms selected from oxygen, nitrogen, and sulfur; provided that, including $S_oE_o$, the carbon atom of $B^3$ bonded to $K^2$ as defined below does not carry ---$CH_2OH$ ----$CHCH_3OH$ or --$C(CH_3)_2$ OH; $K^1$ is hydrogen or is a straight chain or branched chain, cyclic or acyclic, saturated, unsaturated, and/or aromatic carbon- and/or heteroatom-containing group containing not more than 15 carbon atoms, not more than 18 halogen atoms, and not more than 6 heteroatoms selected from oxygen, nitrogen, and sulfur, and $K^1$ may be linked to $B^2$ or $B^3$ or to both $B^2$ and $B^3$ to form one or more additional carbocyclic and/or heterocyclic rings; $K^2$ is selected from oxygen, sulfur --$NK^6$-- or ---$CK^6K^7$-- wherein $K^6$ is hydrogen, hydroxy, methyl, ethyl, fluoro, n-propyl, allyl, or propargyl, and $K^7$ is hydrogen, methyl, ethyl, halogen, trifluoromethyl or cyano; $K^3$ and $K^4$ may be the same or may differ and may each be hydrogen or a straight chain or branched chain, cyclic or acyclic, saturated, unsaturated, and/or aromatic carbon- and/or heteroatom-containing group, such that $K^3$ and $K^4$ taken together contain not more than 18 carbon atoms, not more than 24 halogen atoms, and not more than 6 heteroatoms selected from oxygen, nitrogen and sulfur; $K^5$ is selected from oxygen, sulfur, sulfoxide, sulfone or --$NK^8$--, --$NOK^8$-- or ----$CK^8K^9$---- wherein $K^8$ is hydrogen or a straight chain or branched chain, cyclic or acyclic, saturated, unsaturated, and/or aromatic carbon- and/or heteroatom-containing group containing not more than 30 carbon atoms, not more than 24 halogen atoms, and not more than 8 heteroatoms selected from oxygen, nitrogen, and sulfur, and $K^9$ is hydrogen, methyl, ethyl, n-propyl, hydroxy, halogen, allyl, propargyl, cyano, or trifluoromethyl; provided that if $B^1$ completes an unsubstituted carbocyclic ring, and $B^2$ is ---NH---, and $K^1$ is hydrogen, and $K^2$ is ---NH---, and $K^3$ is ---$CH(CH_3)_2$ in the alpha position, and $K^4$ is hydrogen, and $K^5$ is ---$N(CH_3)$---, then $B^3$ and $S_oE_o$ taken together are not

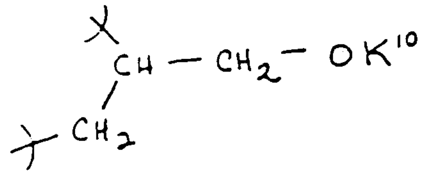

wherein $K^{10}$ is alkyl or alkanoyl.

$P_3$ is a radical of the formula:

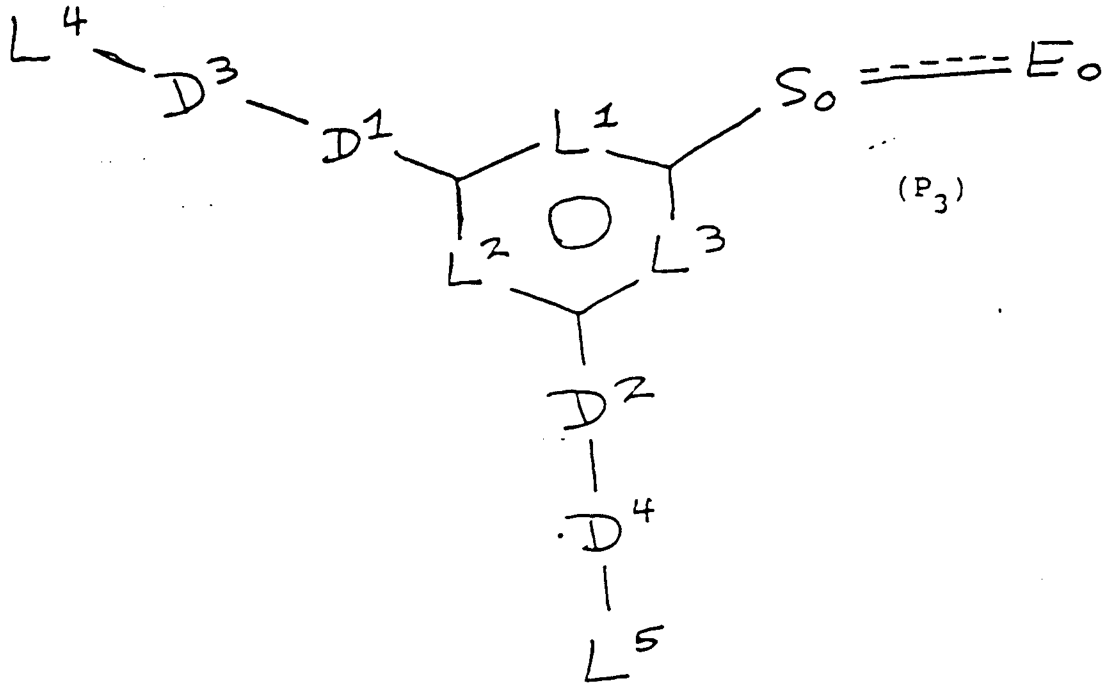

($P_3$)

in the form of an individual isomer, an isomer mixture, a racemate or optical antipode, or a pharmaceutically acceptable salt thereof, wherein

$L^1$, $L^2$ and $L^3$ are individually selected from nitrogen or substituted or unsubstituted carbon;

$D^1$ and $D^2$ each may be a bond or a substituted or unsubstituted carbon atom; $D^1$ may be linked to $L^1$, $L^2$ or both $L^1$ and $L^2$ to form additional fused carbocyclic and/or heterocyclic substituted or unsubstituted rings; and $D^2$ may be linked to $L^2$, $L^3$ or both $L^2$ and $L^3$ to form additional fused carbocyclic and/or heterocyclic substituted or unsubstituted rings; $D^3$ and $D^4$ each are heteroatom-containing functional groups, the heteroatoms being selected from oxygen, nitrogen, and sulfur; $D^3$ may be linked to $L^1$, $L^2$ or both $L^1$ and $L^2$ to form additional fused substituted or unsubstituted carbocyclic and/or heterocyclic rings; and $D^4$ may be linked to $L^2$, $L^3$ or both $L^2$ and $L^3$ to form additional fused substituted or unsubstituted carbocyclic and/or heterocyclic rings; provided that $D^1$ and $D^3$ taken together and $D^2$ and $D^4$ taken together both embody at least one oxygen, nitrogen, or sulfur atom separated from the aromatic nucleus by zero or one intervening carbon atom; and $L^4$ and $L^5$ are straight chain or branched, cyclic or acyclic, saturated, unsaturated, and/or aromatic carbon- and/or heteroatom-containing groups which, taken together, contains about 2-40 carbon atoms, not more than 24 halogen atoms, and not more than 6 heteroatoms selected from oxygen, nitrogen, and sulfur; provided that $S_oE_o$ may not be hydroxymethyl or 1-hydroxyethyl.

$P_4$ is a radical of the formula:

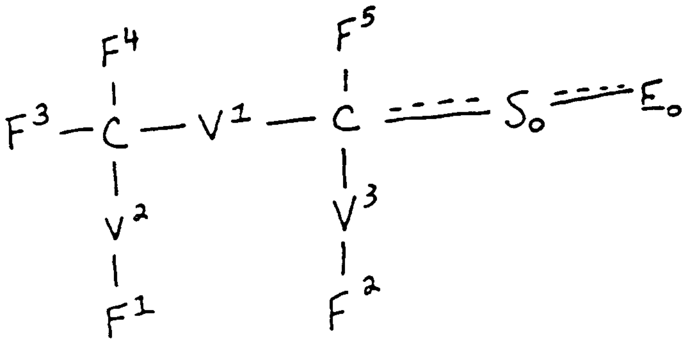

($P_4$)

in the form of an individual isomer, an isomer mixture, a racemate or optical antipode, or a pharmaceutically acceptable salt thereof,

wherein $V^1$ is a bond or is a carbon atom carrying substituents individually selected from hydrogen, methyl, and halogen; $V^2$ and $V^3$ are individually selected from oxygen, sulfur, sulfoxide, and ---$NV^4$--- in which $V^4$ is hydrogen or a hydrocarbon radical containing not more than 30 carbon atoms; $F^1$ and $F^2$ are a staight chain or branched, cyclic or acyclic, saturated, unsaturated, and/or aromatic carbon- and/or heteroatom-containing groups which, taken together, contain 10-40 carbon atoms, not more than 24 halogen atoms, and not more than 8 heteroatoms selected from oxygen, nitrogen, and sulfur; $F^3$ is hydrogen or a substituent selected from methyl, ethyl, trifluoromethyl, 2,2,2-trifluoroethyl, cyano, vinyl, ethynyl, allyl, and propargyl; $F^4$ and $F^5$ each may be hydrogen or may be hydrocarbon or halogenated hydrocarbon radicals which, taken together, contain not more than 40 carbon atoms and not more than 24 halogen atoms; provided that $V^4$, $F^1$, $F^2$, $F^4$ and $F^5$ taken together contain not more than 50 carbon atoms and provided that $S_oE_o$ may not be hydrogen, methyl, chloromethyl, hydroxymethyl, mercaptomethyl, unsubstituted carboxamido, 1-hydroxyethyl or alkanoyloxymethylene.

$P_5$ is a radical of the formula:

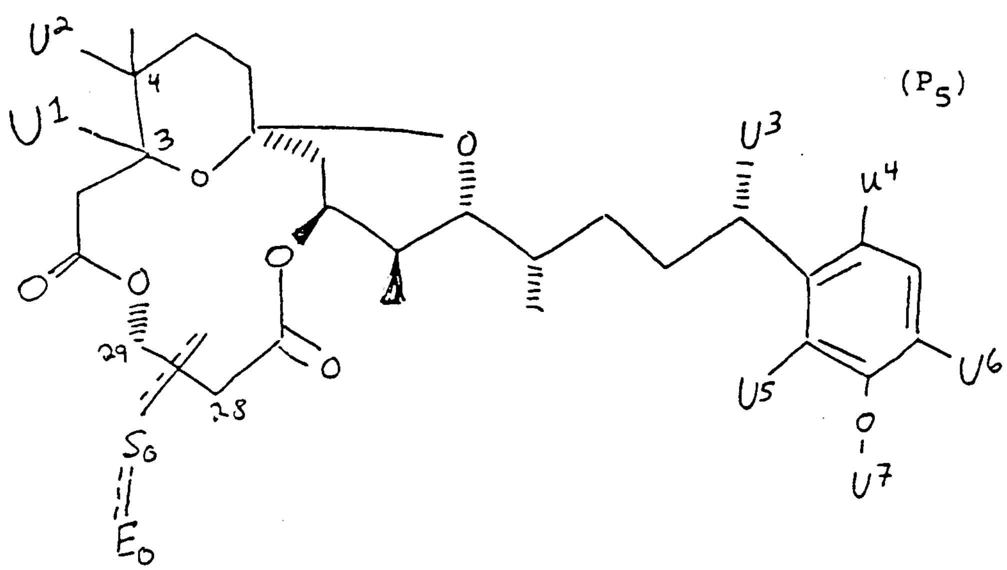

($P_5$)

in the form of an individual isomer, an isomer mixture, a racemate or optical antipode, or a pharmaceutically acceptable salt thereof, wherein $U^1$ and $U^2$, independently, are selected from hydrogen, azide, halogen, hydroxy, $C_{1-7}$ alkoxy, $C_{1-7}$ alkenoxy, $C_{1-7}$ alkynoxy, thiol, $C_{1-7}$ alkanoyl, $C_{1-7}$ saturated or unsaturated alkyl, and cyano; or $U^1$ and $U^2$ taken together may be an oxygen atom forming an epoxy group or may be an additional bond forming an unsaturated linkage; $U^3$ is selected from hydrogen, halogen, $C_{1-12}$ alkyl, $C_1$-$C_{12}$ alkenyl, $C_1$-$C_{12}$ alkynyl, $C_1$-$C_{12}$ alkoxy, $C_1$-$C_{12}$ alkenoxy, $C_1$-$C_{12}$ alkynoxy, and aryl or $C_7$-$C_{12}$ aralkyl wherein the aryl group may be substituted by nitro, halogen, cyano, and/or diloweralkylamino groups; $U^4$-$U^6$, independently, are selected from hydrogen, halogen, cyano, nitro, amino, diloweralkylamino, $C_{1-7}$ saturated or unsaturated alkyl, hydroxy, $C_{1-7}$ saturated or unsaturated alkoxy, $C_{1-7}$ carboalkoxy, $C_{1-7}$ alkanoyloxy,

and azide; and $U^7$ is selected from hydrogen, $C_{1-7}$ saturated or unsaturated alkyl, and $C_{1-7}$ saturated or unsaturated alkanoyl; provided that if $S_oE_o$ is hydroxymethyl, 1-hydroxyethyl or acetoxymethylene, then $S_oE_o$ may not be bonded to C29.

$P_6$ is a radical of the formula:

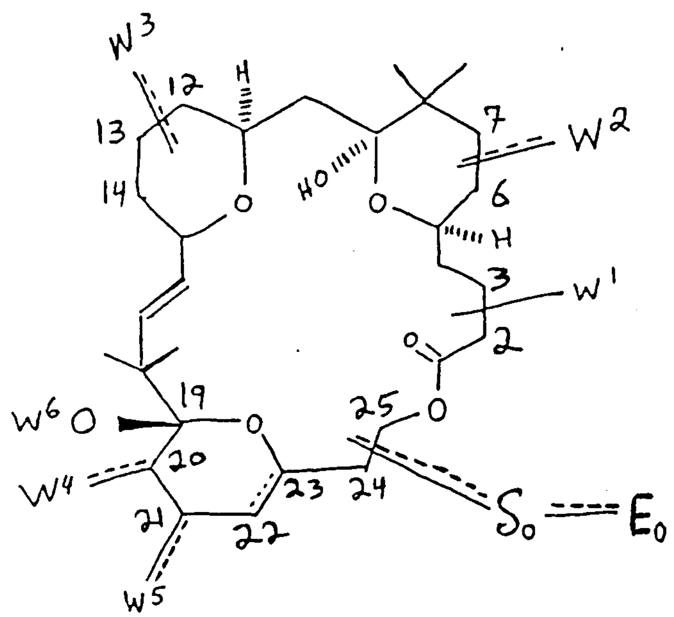

$(P_6)$

in the form of an individual isomer, an isomer mixture, a racemate or optical antipode, or a pharmaceutically acceptable salt thereof,
wherein $W^1$ is selected from hydrogen, halogen, hydroxy, $C_{1-5}$ alkoxy, $C_{1-5}$ alkanoyloxy and cyano; $W^2$ is selected from oxo, hydrogen, hydroxy, cyano, $C_{1-7}$ alkyl, $C_{1-7}$ alkoxy, $C_{1-7}$ alkanoyloxy, and halogen; $W^3$-$W^5$ each may be hydrogen or a straight chain or branched chain, cyclic or acyclic, saturated, unsaturated, and/or aromatic carbon- and/or heteroatom-containing group, containing not more than 30 carbon atoms, not more than 24 halogen atoms, and not more than 8 heteroatoms selected from oxygen, nitrogen, and sulfur, and $W^4$ and $W^5$ taken together may form an additional carbocyclic or heterocyclic ring; and $W^6$ is hydrogen or a straight chain or branched chain, cyclic or acyclic, saturated, unsaturated, and/or aromatic carbon- and/or heteroatom-containing group, containing not more than 15 carbon atoms, not more than 12 halogen atoms and not more than 5 heteroatoms selected from oxygen, nitrogen, and sulfur, and $W^6$, taken together with $W^4$ and $W^5$ may complete an additional carbocyclic or heterocyclic ring; provided that if $S_oE_o$ is hydroxymethyl or hydroxyethyl then $S_oE_o$ may not be bonded to C25.

$S_x$ may represent any of a broad range of connecting chains of atoms, designated $S_1$, $S_2$, $S_3$, $S_4$, $S_5$ and $S_6$. Surprisingly, these chains may be hydrophobic in nature, with few if any polar or heteroatoms present, may be halogen-substituted, or may contain one or several polar atoms such as oxygen, nitrogen, and/or sulfur in any of numerous chemical groupings, and the resultant compounds then display the antagonistic properties described in this invention.

$S_1$ is a single or a double bond or is a chain of atoms of the formula:

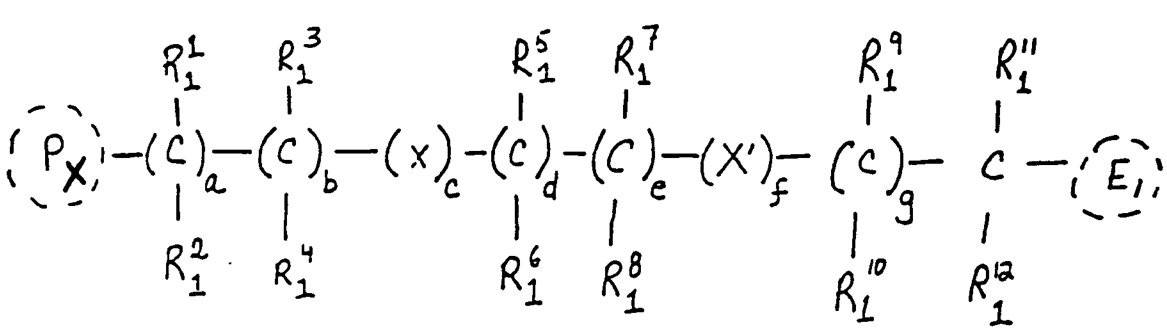

wherein a, b, d, e, and g may independently be from 0 to 3; c and f may independently be 0 or 1; the sum of

(a+b+c+d+e+f+g) is at least 1 but not more than 6; and if c and f are both 1, then the sum of (d+e) must be at least 1; $R_1^1$ through $R_1^{12}$ may be the same or different and each may be either hydrogen or a saturated or singly or multiply unsaturated, straight or branched, acyclic substituent containing not more than 6 carbon atoms, not more than 9 halogen atoms, and not more than 6 heteroatoms selected from oxygen, nitrogen, and sulfur, such that for any substituent the oxygen, nitrogen, and sulfur atoms are situated in functional groups selected from hydroxy, amino, hydroxylamine, tertiary amine oxide, Schiff's base, hydrazine, thiol, nitro, nitroso, oxime, azide, ether, acetal, ketal, thioether, aldehyde, keto, hydrazone, carboxy, ester, amide, cyano, hydrazide, carbonate, carbamate, urea, isourea, carboxamidine, imidate, guanidine, thioester, thioamide, thiocarbonate, dithiocarbonate, thiocarbamate, dithiocarbamate, thiourea, isothiourea, thioimidate, nitroguanidine, cyanoguanidine, and xanthate;
$R_1^1$ or $R_1^2$ may optionally be an additional bond completing an unsaturated linkage to the $P_x$; one of the substituents $R_1^1$ -$R_1^{12}$ may be $G^1$, as defined below, provided that the substituent is bonded to a carbon atom that is separated from the $P_x$ by either zero or one intervening atom; $R_1^{11}$ or $R_1^{12}$ may optionally be an additonal bond to $E_1$, thereby completing an unsaturated linkage; one of the substituents $R_1^1$ -$R_1^{12}$ may be linked to either the atom in $P_x$ that carries the $S_1$ chain or to an atom in $P_x$ adjacent thereto, to form a saturated, unsaturated, or aromatic, carbocyclic or heterocyclic 4-8 membered ring optionally containing 1-4 identical or different ring hetero members selected from O, S, SO, $SO_2$, CO, =N-, and NH, the ring being optionally substituted on any carbon and/or NH members, by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy; X and X' are defined below; provided that, for all substituents $R_1^1$ through $R_1^{12}$ and all constituents and all constituents X and X', taken together, but excluding any atoms of the $P_X$: the total of carbon atoms is 10 or less; the total of halogen atoms is 12 or less; the total of oxygen atoms is 4 or less; the total of nitrogen atoms is 4 or less; the total of sulfur atoms is 3 or less; the total of oxygen, nitrogen, and sulfur atoms together is 8 or less; the total of --OH groups is 3 or less; the total of --$NH_2$ groups is 2 or less; the total of --SH groups is 2 or less; the total of --OH, --SH, and --$NH_2$ groups together is 4 or less.

$S_2$ is a chain of atoms of the formula:

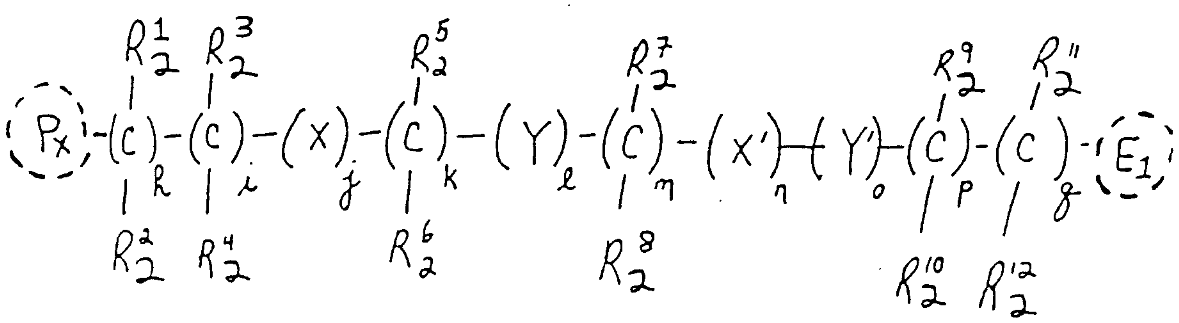

wherein h, i, k, m, p, and q may be independently be from 0 to 3; j and n may independently be 0 or 1; if j and n are both 1 and l is 0, then the sum of (k + m) must be at least 1;
if n is 1 and o is 0, then the sum of (p + q) must be at least 1; the sum of (l + o) is 1-3; and the sum of (h+i+j+k+2l+m+n+2o+p+q) is at least 1 but not more than 7; $R_2^1$ through $R_2^{12}$ may be the same or different and each may be either hydrogen or a saturated or singly or multiply unsaturated, straight or branched, acyclic substituent containing not more than 6 carbon atoms, not more than 9 halogen atoms, and not more than 6 heteroatoms selected from oxygen, nitrogen, and sulfur, such that for any substituent the oxygen, nitrogen, and sulfur atoms must be situated in functional groups selected from hydroxy, amino, hydroxylamine, tertiary amine oxide, Schiff's base, hydrazine, thiol, nitro, nitroso, oxime, azide, ether, acetal, ketal, thioether, aldehyde, keto, hydrazone, carboxy, ester, amide, cyano, hydrazide, carbonate, carbamate, urea, isourea, carboxamidine, imidate, guanidine, thioester, thioamide, thiocarbonate, dithiocarbonate, thiocarbamate, dithiocarbamate, thiourea, isothiourea, thioimidate, nitroguanidine, cyanoguanidine,
and xanthate; $R_2^1$ or $R_2^2$ may optionally be an additional bond completing an unsaturated linkage to $P_x$; one of the substituents $R_2^1$ -$R_2^{12}$ may be $G^1$,as defined below, provided that said substituent is bonded to a carbon atom that is separated from the $P_x$ by either zero or one intervening atom; $R_2^{11}$ or $R_2^{12}$ may optionally be an additional bond to $E_1$, thereby completing an unsaturated linkage; one of the substituents $R_2^1$ -$R_2^{12}$ may be linked to either the atom in $P_x$ that carries the $S_2$ chain or to an atom in $P_x$ adjacent thereto, to form a saturated, unsaturated, or aromatic, carbocyclic or heterocyclic 4-8 membered ring optionally containing 1-4 identical or different hetero ring members selected from O, S, SO, $SO_2$, CO, =N-, and NH, the ring being optionally substituted on any carbon and/or NH members by 1-4 identical or

different substituents selected from halogen, hydroxy, methoxy, ethoxy, methyl, ethyl, cyano, azide, nitro, hydroxymethyl, 1-hydroxyethyl, 2-hydroxy-2propyl, $CF_3$, $OCF_3$, SH, $SCH_3$, $SOCH_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy:
X and X' and Y and Y' are as defined below;
provided that, for all substituents $R_2^1$ through $R_2^{12}$ and all substituents embodied in X, X', Y, and Y' taken together, but excluding any atoms of $P_x$: the total of carbon atoms is 10 or less; the total of halogen atoms is 12 or less; the total of oxygen atoms is 4 or less; the total of nitrogen atoms is 4 or less; the total of sulfur atoms is 3 or less; the total of oxygen, nitrogen, and sulfur atoms together is 8 or less; the total of --OH groups is 3 or less; the total of $--NH_2$ groups is 2 or less; the total of --SH groups is 2 or less; the total of --OH, --SH, and $--NH_2$ groups together is 4 or less.

$S_3$ is a chain of atoms of the formula:

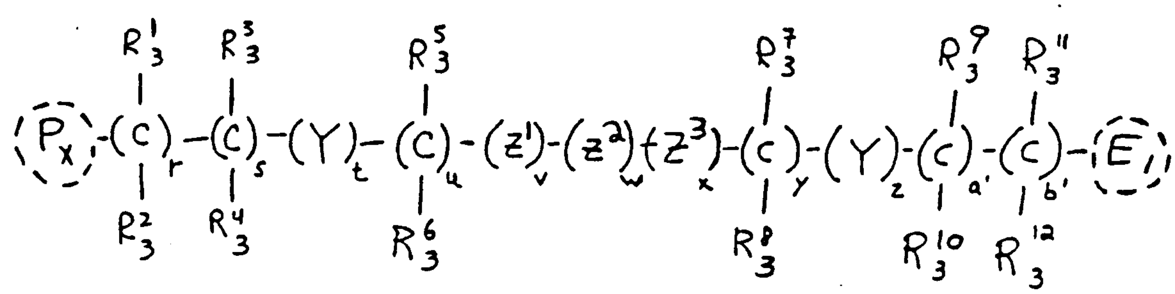

wherein r, s, u, y, a', and b' may independently be from 0 to 3; the sum of (t + z) is 0 or 1; the sum of (v + w + x) is 1; the sum of (y + z + a' + b') is at least 1; and the sum of (r + s + 2t + u + 2v + 3w + 4x + y + 2z + a' + b') is at least 1 but not more than 7; $R_3^1$ through $R_3^{12}$ may be the same or different and each may be either hydrogen or a saturated or singly or multiply unsaturated, straight or branched, acyclic substituent, containing not more than 6 carbon atoms, not more than 9 halogen atoms, and not more than 6 heteroatoms selected from oxygen, nitrogen, and sulfur, such that for any substituent the oxygen, nitrogen, and sulfur atoms must be situated in functional groups selected from hydroxy, amino, thiol, nitro, azide, ether, thioether, aldehyde, keto, carboxy, ester, amide, cyano, carbonate, carbamate, urea, isourea, carboxamidine, guanidine, thioester, thioamide, thiocarbonate, thiocarbamate, thiourea, nitroguanidine, cyanoguanidine, and
xanthate $R_3^1$ or $R_3^2$ may optionally be an additional bond completing an unsaturated linkage to $P_x$; one of the substituents $R_3^1$ -$R_3^{12}$ may optionally be $G^1$, as defined below, provided that said substituent is bonded to a carbon atom that is separated from $P_x$ by either zero or one intervening atom; $R_3^{11}$ or $R_3^{12}$ may optionally be an additional bond to $E_1$, thereby completing an unsaturated linkage; one of the substituents $R_3^1$ -$R_3^{12}$ may be linked to either the atom in $P_x$ that carries the $S_3$ chain or to an atom in $P_x$ adjacent thereto, to form a saturated, unsaturated, or aromatic, carbocyclic 4-8 membered ring optionally containing 1-4 identical or different hetero ring members selected from O, S, SO, $SO_2$, CO, =N-, and NH, the ring being optionally substituted on any carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy;
Y, Y', $Z^1$, $Z^2$, and $Z^3$ are as defined below;
provided that, for all substituents $R_3^1$ through $R_3^{12}$ and all substituents embodied in Y, Y', $Z^1$, $Z^2$, and $Z^3$ taken together, but excluding any atoms of $P_x$: the total of carbon atoms is 10 or less; the total of halogen atoms is 12 or less; the total of oxygen atoms is 4 or less; the total of nitrogen is 4 or less; the total of sulfur atoms is 3 or less; the total of oxygen, nitrogen, and sulfur atoms together is 8 or less; the total of --OH groups is 3 or less; the total of $--NH_2$ groups is 2 or less; the total of --SH groups is 2 or less; the total of --OH, --SH, and $--NH_2$ groups together is 4 or less.

$S_4$ is a chain of atoms defined by:

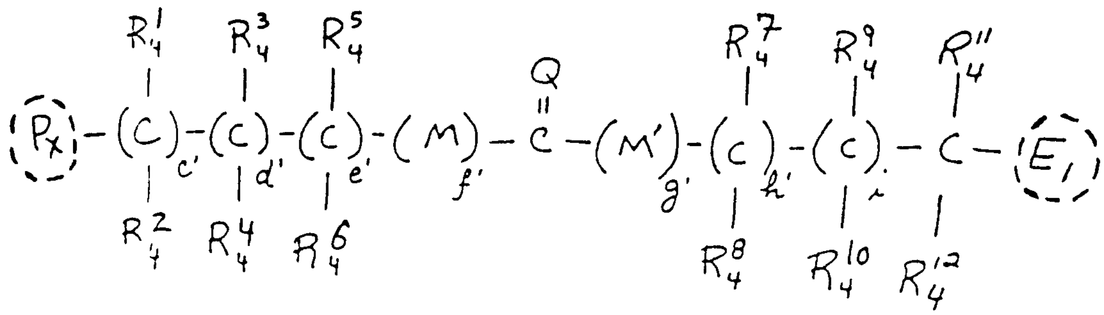

wherein c', d', e', h', and i' may independently be from 0 to 3; f' and g' may independently be 0 or 1; and the sum of (c' + d' + e' + f' + g' + h' + i') is at least 1 but not more than 5; $R_4^1$ through $R_4^{12}$ may be the same or different and each may be either hydrogen or a saturated or singly or multiply unsaturated, straight or branched, acyclic substituent containing not more than 6 carbon atoms, not more than 9 halogen atoms, and not more than 6 heteroatoms selected from oxygen, nitrogen, and sulfur, such that for any substituent the oxygen, nitrogen, and sulfur atoms must be situated in functional groups selected from hydroxy, amino, thiol, nitro, azide, ether, thioether, aldehyde, keto, carboxy, ester, amide, cyano, carbonate, carbamate, urea, isourea, carboxamidine, guanidine, thioester, thioamide, thiocarbonate, thiocarbamate, thiourea, nitroguanidine, cyanoguanidine, and xanthate; $R_4^1$ or $R_4^2$ may optionally be an additional bond completing an unsaturated linkage to $P_x$; one of the substituents $R_4^1$ -$R_4^{12}$ may optionally be $G^1$, as defined below, provided that said substituent is bonded to a carbon atom that is separated from $P_x$ by either zero or one intervening atom; $R_4^{11}$ or $R_4^{12}$ may optionally be an additional bond to $E_1$, thereby completing an unsaturated linkage; one of the substituents $R_4^1$ -$R_4^{12}$ may be linked to either the atom in $P_x$ that carries that $S_4$ chain or to an atom in $P_x$ adjacent thereto, to form a saturated, unsaturated, or aromatic, carbocyclic or heterocyclic 4-8 membered ring optionally containing 1-4 identical or different hetero ring members selected from O, S, SO, $SO_2$, CO, =N-, and NH, the ring being optionally substituted on any carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido, containing 1-4 carbon atoms inclusive, hydroxyacetyl and hydroxyacetoxy; M, M', and Q are as defined below; provided that, for all substituents are $R_4^1$ through $R_4^{12}$ and all constituents M, M', and Q taken together, but excluding any atoms of $P_x$: the total of carbon atoms is 10 or less; the total of halogen atoms is 12 or less; the total of oxygen atoms is 4 or less; the total of nitrogen atoms is 4 or less; the total of sulfur atoms is 3 or less; the total of oxygen, nitrogen, and sulfur atoms together is 8 or less; the total of --OH groups is 3 or less; the total of --$NH_2$ groups is 2 or less; the total of --SH groups is 2 or less; the total of --OH, --SH, and --$NH_2$ groups together is 4 or less.

$S_5$ is a chain of atoms defined by:

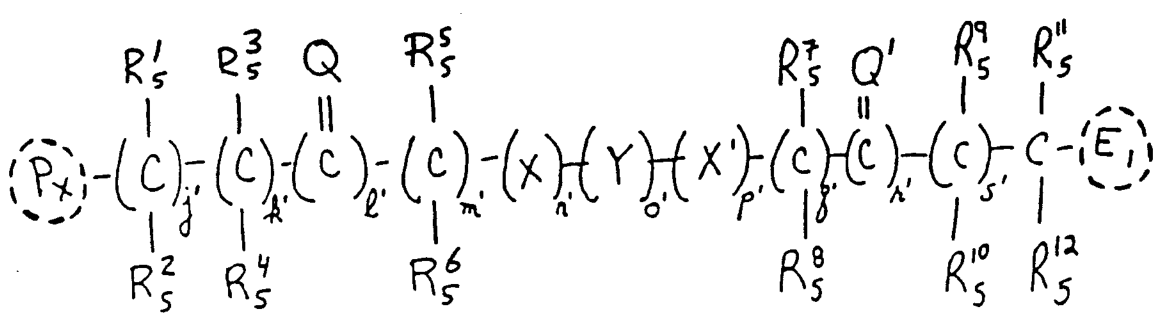

wherein j', k', m', q', and s' may independently be from 0 to 3; l' or r' may each be 0 or 1, but the sum of (l' + r') must be 1 or 2; n' and p' may each be 0 or 1, but the sum of (n' + p')must be 0 or 1; the value of o' may be 0-2; if the sum of (n' + p') is 1 and l' is 0, then q' must be at least 1; if the sum of (n' + p') is 1 and r' is 0, then m' must be at least 1; and the sum of (j' + k' + l' + m' + n' + o' + p' + q' + r' + s') is at least 1 but not more than 5; $R_5^1$ through $R_5^{12}$ may be the same or different and each may be either hydrogen or a saturated or singly or multiply unsaturated, straight or branched, acyclic

substituent containing not more than 6 carbon atoms, not more than 9 halogen atoms, and not more than 5 heteroatoms selected from oxygen, nitrogen, and sulfur, such that for any substituent the oxygen, and sulfur atoms must be situated in functional groups selected from hydroxy, amino, thiol, nitro, azide, ether, thioether, aldehyde, keto, carboxy, ester, amide, cyano, carbonate, carbamate, urea, isourea, carboxamidine, guanidine, thioester, thioamide, thiocarbonate, thiocarbamate, thiourea, nitroguanidine, cyanoguanidine, and xanthate; $R_5^1$ or $R_5^2$ may optionally comprise an additional bond completing an unsaturated linkage $P_x$; one of the substituents $R_5^1$-$R_5^{12}$ may optionally represent $G^1$, as defined below, provided that said substituent is bonded to a carbon atom that is separated from $P_x$ by either zero or one intervening atom; $R_5^{11}$ or $R_5^{15}$ may optionally comprise an additional bond to $E_1$, thereby completing an unsaturated linkage; one of the substituents $R_5^1$-$R_5^{12}$ may be linked to either the atom in $P_x$ that carries the $S_5$ chain or to an atoms in adjacent thereto, to form a saturated, unsaturated, or aromatic, carbocyclic 4-8 membered ring optionally containing 1-4 identical or different hetero ring members selected from O, S, SO, $SO_2$, CO, =N-, and NH, the ring being optionally substituted on any carbon and/or NH members by 1-4 identical or different substituents selected from the halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy; Q, Q', X, X', and Y are as defined below; provided that, for all substituents $R_5^1$ through $R_5^{12}$ and all constituents Q, Q', X, X', and Y taken together, but excluding any atoms of $P_x$: the total of carbon atoms is 10 or less; the total of halogen atoms is 12 or less; the total of oxygen atoms is 4 or less; the total if nitrogen atoms is 4 or less; the total of sulfur atoms is 3 or less; the total of oxygen, nitrogen, and sulfur atoms together is 8 or less; the total of --OH groups is 3 or less; the total of --$NH_2$ groups is 2 or less; the total of --SH groups is 2 or less; the total of --OH, --SH, and --$NH_2$ groups together is 4 or less.

$S_6$ is a chain of atoms defined by:

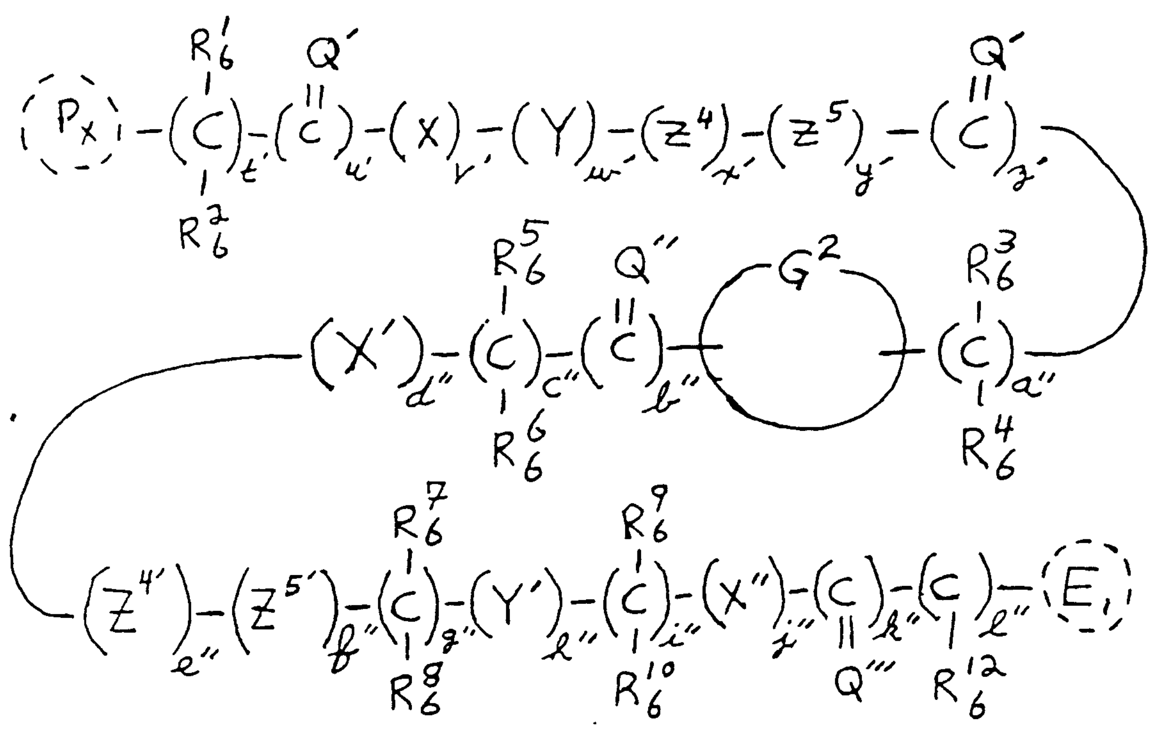

wherein t', u', v', w', x', y', z' and a" may each be 0 or 1; the sum of (t' + u' + v' + 2w' + x' + 2y' + z' + a") must be 0-2; b", d", e", f", h", j" and k" may each independently be 0 or 1; c", g", i", and l" may each independently be 0-3; if d" and j" are both 1, then the sum of (g" + i") must be at least 1; if either j" or k" is 1, then l" must be at least 1; if b" is 1, then the sum of (c" + g" + h" + i" + l") must be at least 1; if d" is 1, then the sum of (g" + h" + i" + l") must be at least 1; and the sum of (t' + u' + v' + 2w' + x' + 2y' + z' + a" + b" + c" + d" + e" + 2f" + g" + 2h" + i" + j" + k" + l") must be 0-5; $R_6^1$ through $R_6^{12}$ may be the same or different and each may be either hydrogen or a saturated or or multiply unsaturated, straight or branched, acyclic substituent containing not more than 6 carbon atoms, not more than 9 halogen atoms, and not more than 5 heteroatoms selected from oxygen, nitrogen, and sulfur, such that for any substituent the oxygen, nitrogen, and sulfur atoms must be situated in functional groups selected from hydroxy, amino, thiol, nitro, azide, ether, thioether, aldehyde, keto, carboxy, ester, amide, cyano, carbonate, carbamate, urea, isourea, carboxamidine, guanidine, thioester, thioamide, thiocarbonate, thiocarbamate, thiourea, nitroguanidine, cyanoguanidine, and xanthate; $R_6^1$ or $R_6^2$

may optionally be an additional bond completing an unsaturated linkage to $P_x$; $R_6^3$ or $R_6^4$ may optionally comprise an additional bond to $G^2$ as defined below, thus completing an unsaturated linkage; $R_6^5$ or $R_6^6$ may optionally comprise an additional bond to $G^2$, thus completing an unsaturated linkage if a" is 0; one of the substituents $R_6^1$ -$R_6^4$ may optionally be $G^1$, as defined below; $R_6^{11}$ or $R_6^{12}$ may optionally comprise an additional bond to $E_1$, thereby completing an unsaturated linkage; one of the substituents $R_6^1$ -$R_6^4$ may be linked to either the atom in $P_x$ that carries the $S_6$ chain or to an atom in $P_x$ adjacent thereto, to form a saturated, unsaturated, or aromatic, carbocyclic 4-6 membered ring optionally containing 1-3 hetero ring members selected from O, S, SO, $SO_2$, CO, =N-, and NH, the ring being optionally substituted on any carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy; Q, Q', Q", Q"', X, X', X", Y, Y', and $Z^4$, are as defined below; provided that, for all substituents $R_6^1$ through $R_6^{12}$ and all constituents Q, Q', Q", Q"', X, X', X", Y, Y', $Z^4$, and $G^2$ taken together, but excluding any atoms of $P_x$: the total of carbon atoms is 12 or less; the total of halogen atoms is 12 or less; the total of oxygen atoms is 5 or less; the total of nitrogen atoms is 4 or less; the total of sulfur atoms is 3 or less, the total of oxygen, nitrogen, and sulfur atoms together is 8 or less; the total of --OH groups is 3 or less; the total of --$NH_2$ groups is 2 or less; the total of --SH groups is 2 or less; the total of --OH, --SH, and --$NH_2$ groups together is 4 or less.

X, X', X" may be the same or different and are selected from:

—O— —S—

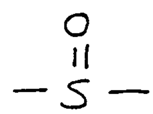

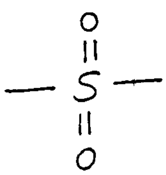

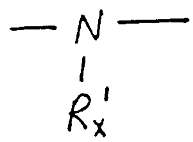

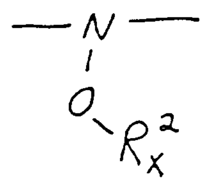

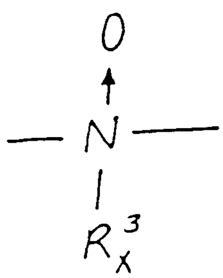

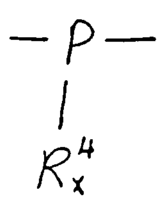

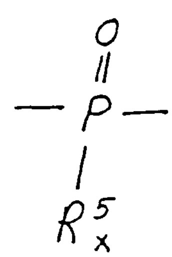

wherein $R_X^1$ and $R_X^2$ may independently be hydrogen;

$R_X^1$ through $R_X^5$ may be the same or different and each may be a saturated or singly or multiple unsaturated, straight or branched, acyclic substituent containing 1-6 carbon atoms, not more than 8 halogen atoms, and not more than 4 heteroatoms selected from oxygen, nitrogen, and sulfur, such that for any substituent the oxygen atoms total 3 or less, the nitrogen atoms total 2 or less, and the sulfur atoms total 1 or zero, the heteroatoms being situated in functional groups selected from hydroxy, amino, thiol, nitro, azide, ether, thioether, aldehyde, keto, carboxy, ester, amide, cyano, carbamate, urea, carboxamidine, guanidine, thioester, thioamide, thiocarbamate, thiourea, nitroguanidine, and cyanoguanidine; $R_X^1$ may $R_X^1$ may optionally represent an additional bond to $P_x$, thus completing an unsaturated linkage; one of the substituents $R_X^1$ -$R_X^5$ may optionally represent $G^1$, as defined below, provided that the substituent is bonded to an atom that is separated from the parent "P" by either zero or one intervening atom; and one of the substituents $R_X^1$ -$R_X^5$ may be linked to either the atom in $P_x$ that carries the chain containing X, X', and/or X" or to another atom in $P_x$ adjacent thereto, to form a saturated, unsaturated or aromatic, heterocyclic 4-8 membered ring optionally containing 1-3 other

identical or different hetero ring members selected from O, S, SO, $SO_2$, CO, =N-, and NH, the ring being optionally substituted on its carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy.

Y and Y' may be the same or different and are selected from:

$-C\equiv C-$,

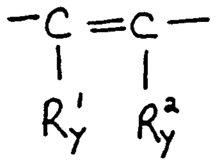

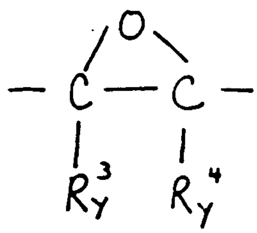

wherein $R_Y^1$ and $R_Y^2$ (cis or trans) may be the same or different and each may be hydrogen or may comprise a saturated or singly or multiply unsaturated, straight or branched acyclic substituent containing not more than 6 carbon atoms, not more than 9 halogen atoms, and not more than 4 heteroatoms selected from oxygen, nitrogen, and sulfur, such that for any substituent the oxygen atoms total 3 or less, the nitrogen atoms total 2 or less, and the sulfur atoms total 1 or zero, the heteroatoms being situated in functional groups selected from hydroxy, amino, thiol, nitro, azide, ether, thioether, aldehyde, keto, carboxy, ester, amide, cyano, carbamate, urea, carboxamidine, guanidine, thioester, thioamide, thiocarbamate, thiourea, nitroguanidine, and cyanoguanidine; $R_Y^3$ and $R_Y^4$ (cis or trans) may be the same or different and each may be hydrogen or may be a saturated or singly or multiply unsaturated, straight or branched acyclic substituent containing not more than 6 carbon atoms, not more than 9 halogen atoms, and not more than 4 heteroatoms selected from oxygen, nitrogen, and sulfur, such that for any substituent the oxygen atoms total 3 or less, the nitrogen atoms total 2 or less, and the sulfur atoms total 1 or zero, the heteroatoms being situated in functional groups selected from hydroxy, amino, thiol, nitro, azide, ether, thioether, aldehyde, keto, carboxy, ester, amide, cyano, carbamate, urea, carboxamidine, guanidine, thioester, thioamide, thiocarbamate, thiourea, nitroguanidine, and cyanoguanidine, with the exception that neither $R_Y^3$ nor $R_Y^4$ may be a halogen atom alone; one of the substituents $R_Y^1$-$R_Y^4$ may optionally represent $G^1$, as defined below, provided that the substituent is bonded to an atom that is separated from $P_x$ by either zero or one intervening atom; and one of the substituents $R_Y^1$-$R_Y^4$ may be linked to either the atom in $P_x$ that carries the chain containing Y and/or Y' or to an atom in adjacent thereto, to form a saturated, unsaturated, or aromatic, carbocyclic 4-8 membered ring optionally containing 1-4 identical or different hetero ring members selected from O, S, SO, $SO_2$, CO, =N-, and NH, the ring being optionally substituted on its carbon and/or NH memebers by 1-4 identical or different substituents selected from halogen; hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy.

$Z^1$ is selected from:

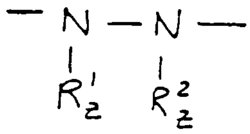
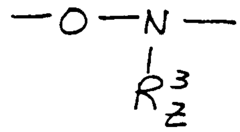
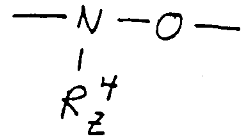

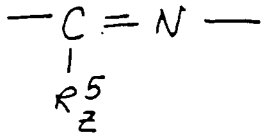
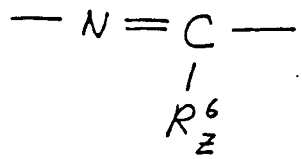

wherein $R_Z^1$ is hydrogen, a saturated or unsaturated substituent selected from $C_{1-6}$ alkyl, $C_{1-6}$ halogenated alkyl, $C_{1-6}$ acyl, $C_{1-6}$ halogenated acyl, $C_{1-6}$ monohydroxyacyl, and $C_{2-6}$ hydroxyalkyl, cyclohexyl, or phenyl or benzyl optionally substituted by methyl, ethyl, methoxy, nitro, cyano, trifluoromethyl, and/or halogen, or, $R_Z^1$ may comprise an additional bond to $P_x$, thus completing an unsaturated linkage; $R_Z^2$ is hydrogen, a saturated or unsaturated substituent selected from $C_{1-6}$ alkyl, $C_{1-6}$ halogenated alkyl, $C_{1-6}$ acyl, $C_{1-6}$ halogenated acyl, $C_{1-6}$ monohydroxyacyl, and $C_{2-6}$ hydroxyalkyl, cyclohexyl, or phenyl or benzyl optionally substituted by methyl, ethyl, methoxy, nitro, cyano, trifluoromethyl, and/or halogen;

$R_Z^3$ independently may have the values specified above for $R_Z^2$ ; $R_Z^4$ independently may have the values specified above for $R_Z^1$ ; $R_Z^5$ may be hydrogen or a saturated or unsaturated substituent selected from $C_{1-6}$ alkyl, $C_{1-6}$ hydroxyalkyl, $C_{1-6}$ alkoxy, $C_{1-6}$ hydroxyalkoxy, $C_{1-6}$ halogenated alkyl, or phenyl or benzyl optionally substituted by methyl, ethyl, methoxy, nitro, cyano, trifluoromethyl, and/or halogen; $R_Z^6$ independently may have the values specified above for $R_Z^5$ ; one of the substituents $R_Z^1$ -$R_Z^6$ may be linked to either the atom in $P_x$ that carries the chain containing $Z^1$ or to atom in $P_x$ adjacent thereto, to form a saturated, unsaturated, or aromatic, carbocyclic or heterocyclic 4-8 membered ring optionally containing 1-3 other identical or different hetero ring members selected from O, S, =N-, and NH, the ring being optionally substituted on its carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy; provided that only one of the substituents $R_Z^1$ -$R_Z^6$ may be substituted or unsubstituted phenyl or benzyl.

$Z^2$ is selected from:

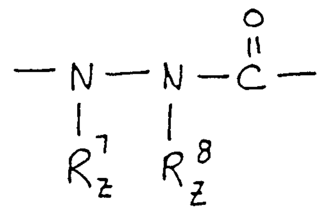

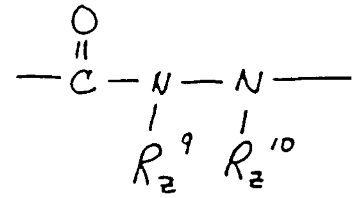

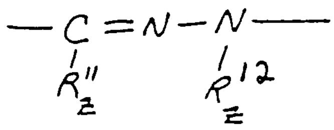

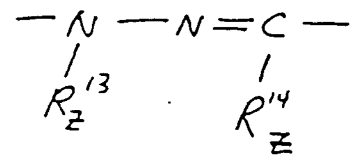

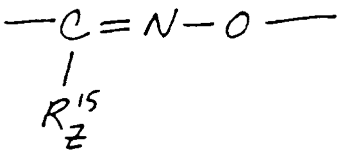

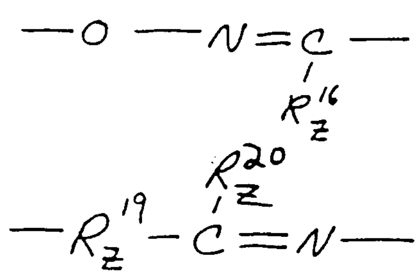

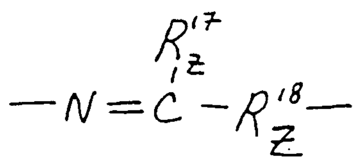

wherein $R_Z^7$ independently may have the values specified for $R_Z^1$ ; $R_Z^8$ is hydrogen, a saturated or unsaturated substituent selected from $C_{1-6}$ alkyl, $C_{1-6}$ halogenated alkyl, $C_{2-6}$ hydroxyalkyl in which the hydroxy group may be esterified to the acetyl or propionyl ester, and cyclohexyl, or phenyl or benzyl optionally substituted by methyl, ethyl, nitro, cyano, trifluoromethyl, and/or halogen; $R_Z^9$ independently may have the values specified above above for $R_Z^8$ ; $R_Z^{10}$ independently may have the values specified above for $R_Z^7$; $R_Z^{11}$ may be hydrogen, a saturated or unsaturated substituent selected from $C_{1-6}$ alkyl, $C_{1-6}$ halogenated alkyl, and cyclohexyl, or phenyl or benzyl optionally substituted by methyl, ethyl, nitro, cyano, trifluoromethyl, and/or halogen; $R_Z^{12}$ -$R_Z^{13}$ independently may have the values specified above for $R_Z^2$ ; $R_Z^{14}$ -$R_Z^{16}$ independently may have the values specified above for $R_Z^{11}$ ; $R_Z^{17}$ and $R_Z^{20}$ individually may be hydrogen or a substituent selected from $C_{1-4}$ alkyl; $C_{1-4}$ alkoxy; $C_{1-4}$ alkylthio, phenoxy or thiophenoxy optionally substituted by methyl, halogen, and/or nitro; or amino optionally mono- or disubstituted by $C_{1-4}$ alkyl or monosubstituted by cyano, nitro, or phenyl optionally substituted by halogen and/or nitro; $R_Z^{18}$ and $R_Z^{19}$ individually may be --O--, --S--, or --N$R_Z^{21}$ , wherein $R_Z^{21}$ may be hydrogen, $C_{1-4}$ alkyl, 2-hydroxyethyl, 2-hydroxy-n-propyl, 2-acetoxyethyl, or 2-acetoxy-n-propyl; one of the substituents $R_Z^7$ -$R_Z^{17}$ , $R_Z^{20}$ or $R_Z^{21}$ optionally may be linked to either the atom in $P_x$ that carries the chain containing $Z^2$ to an atom in $P_x$ adjacent thereto, to form a saturated, unsaturated, or aromatic, carbocyclic or heterocyclic 4-8 membered ring optionally containing 1-3 other identical or different hetero ring members selected from O, S, =N-, and NH, the ring being optionally substituted on its carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive hydroxyacetyl and hydroxyacetoxy; provided that only one of the substituents $R_Z^7$ -$R_Z^{21}$ may comprise or carry a substituted or unsubstituted phenyl or benzyl.

$Z^3$ is selected from:

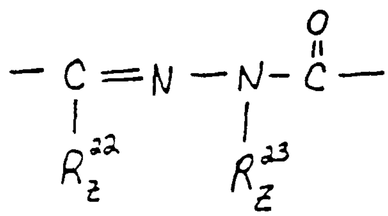

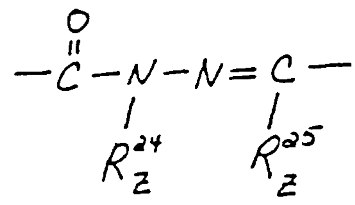

wherein $R_Z^{22}$ and $R_Z^{25}$ independently may have the values specified above for $R_Z^{11}$ ; $R_Z^{23}$ and $R_Z^{24}$ independently may have the values specified above for $R_Z^{8}$ ; $R_Z^{22}$ or $R_Z^{25}$ optionally may be linked to either the atom in $P_x$ that carries the chain containing $Z^2$ or to an atom in $P_x$ adjacent thereto, to form a saturated, unsaturated, or aromatic, carbocyclic or heterocyclic 4-8 membered ring optionally containing 1-3 other identical or different hetero ring members selected from O, S, =N-, and NH, the ring being optionally substituted on its carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive acetamido, N-methylacetamido, carboxamido, N-alkylated carboxamido containing 1-4 carbon atoms inclusive, hydroxyacetyl and hydroxyacetoxy; provided that at most one of the substituents $R_Z^{22}$ -$R_Z^{25}$ may comprise or carry a substituted or unsubstituted phenyl or benzyl moiety.

$Z^4$ and $Z^{4'}$ independently may be:

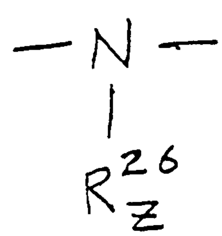

wherein $R_Z^{26}$ may be hydrogen or may be a saturated or singly or multiply unsaturated, straight or branched, acyclic substituent containing 1-6 carbon atoms, not more than 8 halogen atoms, and not more than 4 heteroatoms selected from oxygen, nitrogen, and sulfur, in which substituent the oxygen atoms total 3 or less, the nitrogen atoms total 2 or less, and the sulfur atoms total 1 or zero, the heteroatoms being situated in functional groups selected from hydroxy, amino, thiol, nitro, azide, ether, thioether, aldehyde, keto, carboxy, ester, amide, cyano, carbamate, urea, carboxamidine, guanidine, thioester, thioamide, thiocarbamate, thiourea, nitroguanidine, and cyanoguanidine; $R_Z^{26}$ may optionally comprise one additional bond either to the parent $P_x$ or to $G^2$, thus completing an unsaturated linkage; $R_Z^{26}$ may be linked to either the atom in $P_x$ that carries the $S_6$ chain or to an atom in $P_x$ adjacent thereto, to form a saturated, unsaturated, or aromatic, carbocyclic or heterocyclic 4-6 membered ring optionally containing 1-3 identical or different ring members selected from O, S, SO, $SO_2$, CO, =N-, and NH, the ring being optionally substituted on its carbon and/or NH members by 1-4 identical or different substitutents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy.

$Z^5$ and $Z^{5'}$ independently may be:

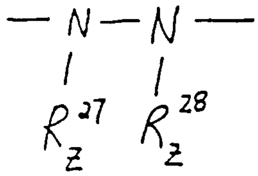

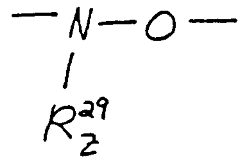

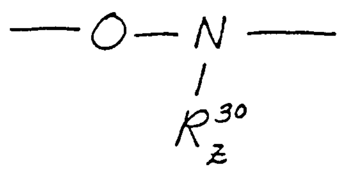

wherein $R_Z^{27}$ is hydrogen, a saturated or unsaturated substituent selected from $C_{1-6}$ alkyl, $C_{1-6}$ halogenated alkyl, $C_{1-6}$ acyl, $C_{1-6}$ halogenated acyl, $C_{1-6}$ monohydroxyacyl, and $C_{2-6}$ hydroxyalkyl, cyclohexyl, or phenyl or benzyl optionally substituted by methyl, ethyl, nitro, cyano, trifluoromethyl, and/or halogen, or $R_Z^{27}$ may comprise an additional bond to the parent $P_x$ or to $G^2$, thus completing an unsaturated linkage; $R_Z^{28}$ is hydrogen, a saturated or unsaturated substituent selected from $C_{1-6}$ alkyl, $C_{1-6}$ halogenated alkyl, $C_{1-6}$ acyl, $C_{1-6}$ halogenated acyl, $C_{1-6}$ monohydroxyacyl, and $C_{2-6}$ hydroxyalkyl, cyclohexyl, or phenyl or benzyl either of which may optionally substituted by methyl, ethyl, nitro, cyano, trifluoromethyl, and/or halogen, or $R_Z^{28}$ may comprise an additional bond to $G^2$, thus completing an unsaturated linkage; $R_Z^{29}$ independently may have the values specified above for $R_Z^{27}$ ; and $R_Z^{30}$ independently may have the values specified above for $R_Z^{1}$.

M and M' independently may be:

—O— —S—

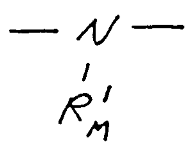

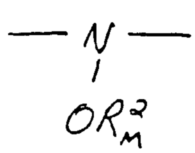

wherein $R_M^1$ and $R_M^2$ may be the same or different and each may be hydrogen or a saturated or singly or multiply unsaturated, straight or branched, acyclic substituent containing 1-6 carbon atoms, not more than 8 halogen atoms, and not more than 4 heteroatoms selected from oxygen, nitrogen, and sulfur, in which the oxygen atoms total 3 or less, the nitrogen atoms total 2 or less, and the sulfur atoms total 1 or zero, the heteroatoms being situated in functional groups selected from hydroxy, amino, thiol, nitro, azide, ether, thioether, aldehyde, keto, carboxy, ester, amide, cyano, nitroguanidine, and cyanoguanidine; $R_M^1$ may optionally comprise an additional bond to the parent $P_x$ group, thus completing an unsaturated linkage;

$R_M^1$ or $R_M^2$ may optionally represent $G^1$, as defined below, provided that said substituent is bonded to an atom that is separated from the parent $P_x$ be either zero or one intervening atoms; $R_M^1$ or $R_M^2$ may be linked to either the atom in $P_x$ that carries the chain containing M and/or M' or to an atom in $P_x$ adjacent thereto, to form a saturated, unsaturated,

or aromatic, carbocyclic 5-8 membered ring optionally containing 1-4 identical or different hetero ring members selected from O, S, CO, =N-, and NH, the ring being optionally substituted on its carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy.

Q-Q''' independently may be:

=O =S =N—$R_Q^1$

=N—O—$R_Q^2$

wherein $R_Q^1$ and $R_Q^2$ may be the same or different and each may be hydrogen or a saturated or singly or multiply unsaturated, straight or branched, acyclic substituent containing 1-6 carbon atoms, not more than 8 halogen atoms, and not more than 4 heteroatoms selected from oxygen, nitrogen, and sulfur, such that for any substituent the oxygen atoms total 3 or less, the nitrogen atoms total 2 or less, and the sulfur atoms total 1 or zero, the heteroatoms being situated in functional groups selected from hydroxy, amino, thiol, nitro, ether, thioether, carboxy, ester, amide, cyano, nitroguanidine, and cyanoguanidine; $R_Q^1$ may optionally represent $G^1$, as defined below, provided that $R_Q^1$ is bonded to an atom that is separated from the parent $P_x$ by either zero or one intervening atom; $R_Q^1$ may be linked to either the atom in $P_x$ that carries the chain containing Q and/or Q' or to an atom in $P_x$ adajcent thereto, to form a saturated, unsaturated, or aromatic, carbocyclic 5-8 membered ring optionally containing 1-4 identical or different hetero ring memebers selected from O, S, CO, =N-, and NH, the ring being optionally substituted on its carbon and/or NH members by 1-4 identical or different substituents selected from halogen, hydroxy, methoxy, ethoxy, methyl, ethyl, cyano, azide, nitro, hydroxymethyl, 1-hydroxyethyl, 2-hydroxy-2-propyl, $CF_3$, $OCF_3$, SH, $SCH_3$, $SOCH_3$, $SCF_3$, COOH, $COOCH_3$, CH=O, acetoxy, amino mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy; $G^1$ comprises a saturated, unsaturated, or aromatic, carbocyclic or heterocyclic 4-8 membered ring optionally containing 1-4 identical or different hetero ring members selected from O, S, SO, $SO_2$, CO, =N-, and NH, the ring being optionally substituted on its carbon and/or NH members by 1-4 identical substituents selected from halogen, hydroxy, methoxy, ethoxy, methyl, ethyl, cyano, azide, nitro, hydroxymethyl, 1-hydroxyethyl, 2-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methyl acetamido, carboxamido, N-alkylated carboxamido containing 1-4 carbon atoms inclusive, hydroxyacetyl and hydroxyacetoxy; $G^2$ completes a saturated, unsaturated, or aromatic, carbocyclic or heterocyclic 4-8 membered ring optionally containing 1-4 identical or different hetero ring members selected from O, S, SO, $SO_2$, CO, =N-, and NH, the ring being optionally substituted on its carbon and/or NH members by 1-4 identical substituents selected from halogen, hydroxy, methoxy, ethoxy, methyl, ethyl, cyano, azide, nitro, hydroxymethyl, 1-hydroxyethyl, 2-hydroxyethyl, 2-hydroxy-2-propyl, $CF_3$, $OCF_3$, SH, $SCH_3$, $SOCH_3$, $SCF_3$, COOH, $COOCH_3$, $COCH_3$, CH=O, acetoxy, amino, mono- or dialkylamino totalling 1-4 carbon atoms inclusive, acetamido, N-methylacetamido, carboxamido, N-alkylated carboxamido containing 1-4 carbon atoms inclusive, hydroxyacetyl and hydroxyacetoxy, and the ring may be connected directly by single or double bonds to adjacent atoms.

The capping group $E_o$ that terminates the connecting chain also may be selected from any of a surprisingly broad array of chemical groupings. These may include, without limitation, hydrophobic entities such as alkyl, hydrogen, and halogenated alkyl, or may include, without limitation, quite hydrophilic moieties, such as hydroxy, thiol, carboxy and carboxy esters, amines, etc. Indeed, the single restriction appears to be that $S_oE_o$ taken together should not be hydroxymethyl or 1-hydroxyethyl bonded in the usual position in the parent compounds, since such compounds correspond to the toxic parent natural products.

Thus, $E_o$ may be a moiety $E_1$, wherein $E_1$ is selected from =O, =S, =NH, =NOH, =N--$NH_2$, hydrogen, halogen, --OH, --SH, --$NH_2$, --NH--$NH_2$, --$N_3$, --NO, --$NO_2$, --NHOH, --$ONH_2$, or is selected from :

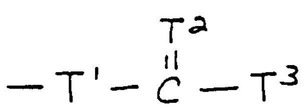 . 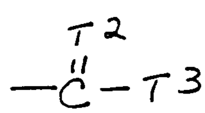

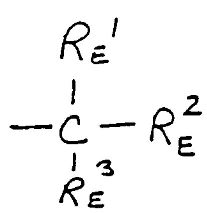

=$CR_E^4R_E^5$ wherein $T^1$ is selected from --O--, --S--, and --NH--; $T^2$ is selected from =O, =S, and =N--$R_E^6$ , in which $R_E^6$

may be hydrogen, cyano, or nitro; $T^3$ is selected from hydrogen, --OH, --$NH_2$, --SH, $N_3$, --NH--$NH_2$, and --NH--$OR_E^7$, in which $R_E^7$ may be hydrogen $C_{1-3}$, alkyl, or $C_{1-3}$ acyl; $R_E^1$ is selected from hydrogen, halogen, hydroxy, nitro, nitroso, cyano, azide, --$NH_2$, --NH--OH, --SH, --O--$NH_2$, --NH--$NH_2$, --$T^1$--C=$T^2$--$T^3$, and --C=$T^2$--$T^3$; $R_E^2$ and $R_E^3$ are individually selected from hydrogen, --C=$T^2$--$T^3$, cyano, nitro, azide, and halogen; and $R_E^4$ and $R_E^5$ are individually selected from hydrogen, halogen, cyano, nitro, --C=$T^2$--$T^3$, --$T^1$--C=$T^2$--$T^3$, and --$CR_E^1R_E^2R_E^3$.

In addition to the compounds described above, we have also found that compounds of the diterpene class, with the usual hydroxymethyl or 1-hydroxyethyl group intact and having at least on substituent other than hydrogen at C12 and a hydroxy, amino, thiol, hydroxymethyl, mercaptomethyl, aminomethyl, 2-hydroxyethyl, carboxy, unsubstituted carboxamido, or unsubstituted aminocarbonyloxy group in the alpha configuration at C13, display useful properties as anti-inflammatory agents. Thus, in this very unusual case a minor change in the diterpene moiety at a location other than the hydroxymethyl leads to a very substantial and useful change in biological properties. This is in contrast to the belief that diterpenes such as phorbol 12-decanoate, 12-dodecanoate and 12-myristate are biologically inactive, and it is especially in contrast to the usual association of the hydroxymethyl or 1-hydroxyethyl groups with pro-inflammatory activity.

The compounds of this invention have been found to possess valuable pharmacological properties. They block inflammation, block proliferation of cancer cells, and induce production of thrombolytic activity in human and veterinary medicine. These effects can be demonstrated, for example, by use of standard mouse ear inflammation tests by established agonists such as PMA and the ionophore A23187, by the inhibition of proliferation of human cancer cells in culture by induction of differentiation, and by measurement of fibrinolytic activity in cultured cells.

These compounds also show selective effects as antagonists or protein kinase C in some cases, as noninflammatory agonists for protein kinase C in other cases, and as selective ligands for protein kinase C and/or for phorboid receptors.

Thus, these compounds can be used as agents for the abrogation of pathophysiological conditions and disease states in applications such as anti-inflammatory, anti-psoriatic, anti-cancer, anti-ulcer, anti-hypertensive, anti-asthma, anti-arthritic, anti-autoimmune, anti-nociceptive, anti-secretory, anti-parasitic, anti-amoebic, anti-HTLV-III/LAV viral replication, and any other application in which pathological involvement of protein kinase C is found.

Furthermore, the non-toxic agonists among the compounds of this invention may be used to achieve desired physiological results such as interferon release, interleukin induction, tumor necrosis factor production, immune system stimulation and/or reconstitution, insulin secretion, insulinomimetic activity, acceleration of wound healing, improvement in central nervous system functions such as memory and learning and abrogation of the symptoms or progress of Alzheimer's disease, and any other application for which desirable actions of protein kinase C are found.

As receptor subtype- and/or protein kinase C substype-selective ligands, the compounds of this invention also have very valuable application as experimental agents for research into the role of protein kinase C and/or phorboid receptors in important biological processes and in human and veterinary diseases. Thus, their value extends to their use as pharmacological tools for in vitro and in vivo research, in a manner similar to the important roles that selection agonists and antagonists have played in the studies of the mechanism of action of adrenergic, dopaminergic, opiate, benzodiaepine, cholinergic, and serotoninergic receptor systems, among others.

In addition, the compounds can be used in in vitro diagnostics (e.g., in an assay for protein kinase C). They are also useful as intermediates in the production of other drugs, e.g., as described in the present invention.

The compounds of this invention are generally administered to animals, including but not limited to fish, avians, and mammals including humans.

The pharmacologically active compounds of this invention can be processed in accordance with conventional methods of galenic pharmacy to produce medicinal agents for administration to patients, e.g., mammals including humans.

The compounds of this invention can be employed in admixture with conventional excipients, i.e., pharmaceutically acceptable organic or inorganic carrier substances suitable for parenteral, enteral (e.g., oral) or topical application which do not deleteriously react with the active compounds. Suitable pharmaceutically acceptable carriers include but are not limited to water, salt solutions, alcoholcs, gum arabic, vegetable oils, benzyl alcohols, polyethylene glycols, gelatine, carbohydrates such as lactose, amylose or starch, magnesium stearate, talc, silicic acid, viscous paraffin, perfume oil, fatty acid esters, hydroxy methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds. They can also be combined where desired with other active agents, e.g., enzyme inhibitors, to reduce metabolic degradation.

For parenteral application, particularly suitable are injectable, sterile solutions, preferably oily or aqueous solutions, as well as suspensions, emulsions, or implants, including suppositories. Ampoules are convenient unit dosages.

For enteral application, particularly suitable are tablets, dragees, liquids, drops, suppositories, or capsules. A syrup, elixir, or the like can be used wherein a sweetened vehicle is employed.

Sustained or directed release compositions can be formulated, e.g., liposomes or those wherein the active compound is protected with differentially degradable coatings, e.g., by microencapsulation, multiple coatings, etc. It is also possible to freeze-dry the new compounds and use the lyophilizates obtained, for example, for the preparation of products for injection.

For topical application, there are employed as nonsprayable forms, viscous to semi-solid or solid forms comprising a carrier compatible with topical application and having a dynamic viscosity compatible with topical application and preferably greater than water. Suitable formulations include but are not limited to solutions, suspensions, emulsions, creams, ointments, powders, liniments, salves, aerosols, etc., which are, if desired, sterilized or mixed with auxiliary agents, e.g., preservatives, stabilizers, wetting agents, buffers or salts for influencing osmotic pressure, etc. For topical application, also suitable are sprayable aerosol preparations wherein the active ingredient, preferably in combination with a solid or liquid inert carrier material, is packaged in a squeeze bottle or in admixture with a pressurized volatile, normally gaseous propellant, e.g., a freon.

Generally, the compounds of this invention are dispensed in unit dosage form comprising 0.01 to 1000 mg in a pharmaceutically acceptable carrier per unit dosage. They are incorporated in topical formulations in concentrations of about 0.01 to 10 weight percent.

It will be appreciated that the actual preferred amounts of active compound in a specific case will vary according to the specific compound being utilized, the particular compositions formulated, the mode of application, and the particular sites and organism being treated. Dosages for a given host can be determined using conventional considerations, e.g., by customary comparison of the differential activities of the subject compounds and of a known agent, e.g., by means of an appropriate, conventional pharmacological protocol.

Starting materials for the synthesis of the compounds of this invention may be obtained from any of a wide variety of natural sources, as described in the literature for the diterpenes (R. Schmidt and E. Hecker, Fortschritte D. Chemie Organischer Naturstoffe 31 377-467 (1974) and F. J. Evans and C. J. Soper, Lloydia 41 193-233 (1978), and references cited therein), and indole alkaloids and aplysiatoxins (T. Sugimura, Gann 73 499-501 (1982), and references cited therein). Furthermore, the diterpene, indole alkaloid, diacylglycerol, and diaminobenzyl alcohol compounds are available by synthesis de novo (See Y. Endo et al., Chem. Pharm. Bull. 32 358-361 (1984), Tetrahedron 42, 5905-5924 (1986), and references cited therein; P. A. Wender, Am. Chem. Soc. National Meeting, Chicago, IL, 9 September 1985, Abstract #7, and P.A. Wender et al. Proc. Nat. Acad. Sci. 83, 4214-4218, 1986).

Given starting phorboids containing hydroxymethyl or 1-hydroxyethyl groups, the means for modifying the hydroxymethyl or 1-hydroxyethyl group to produce the compounds of this invention will be obvious to workers with ordinary skill in synthetic organic chemistry. The hydroxymethyl or 1-hydroxyethyl groups may be modified once other regions of the molecule have been suitably protected, using certain favorable methodologies. Once the phorboid nucleus is protected, the hydroxymethyl/1-hydroxyethyl may for example be conveniently capped under very mild conditions by reaction with a substituted or unsubstituted alkyl, aryl, or aralkyl isocyanate in the presence of a catalyst such as dibutyltin dilaurate. The resulting compounds lack the toxic inflammatory activity of the phorboid from which they were derived, and have themselves anti-inflammatory utility.

Conversion of the hydroxy group to a halogen or pseudohalogen not only in itself provides active anti-inflammatory compounds, but also permits displacement of the resultant electrophile by a very wide range of nucleophiles. Particular examples, with limitation, would be reaction with ammonia, methylamine, sodium cyanide, N-methyl-2-hydroxyethylamine, 1[H]-tetrazole, or with the sodium salt of 2-mercaptoethanol, 3-mercaptopropanol, or of 2-hydroxymethylphenol. Many variations may be executed, as described in standard textbooks of synthetic organic chemistry, such as J. March, Advanced Organic Chemistry, Third Edition, Wiley-Interscience New York, 1985.

For those phorboids wherein the hydroxy of the hydroxymethyl group is allylic, such as phorbol, ingenol, and resiniferonol esters, the replacement of the hydroxy by chloro or preferably by bromo or iodo yields compounds that can be conveniently homologated by reaction with activated zinc in the presence of an electrophile such as, without limitation, an aldehyde, ketone, epoxide, or oxetane; the resultant compounds have one or more methylenes inserted between the original hydroxy group and the methylene to which it was attached. An illustration of this would be the reaction of 20-deoxy-20-chlorophorbol 12-myristate 13-acetate with an excess of formaldehyde and an excess of zinc in the presence of tetrahydrofuran and saturated ammonium chloride solution with vigorous stirring for 24 hours. The resultant 20-homophorbol 12-myristate 13-acetate carries a 2-hydroxyethyl group attached to C6 instead of the usual hydroxymethyl group, and has good anti-inflammatory activity.

Alternatively, the hydroxy group in question may be oxidized to an aldehyde or keto group and then reacted via Wittig chemistry to obtain chain-extended compounds terminated by many different capping groups. In particular, a phorbol 12,13-diester may be selectively protected at the 4 and 9 hydroxy groups using trimethylsilyl trifluoromethanesulfonate, followed by reaction with manganese dioxide to obtain a protected aldehyde. The latter compound may be successfully treated with strong Wittig reagents such as the lithium salt of 2-hydroxyethylidenetriphenylphosphorane, followed by deprotection with tetrabutylammonium fluoride to obtain the corresponding chain-extended, 20,21-didehydro-21,22-dihomophorbol diester.

The hydroxymethyl group of suitable phorboids may be oxidized to the carboxylic acid level by methods well-known in the art, and this carboxylic group may be activated for condensation reactions by any of a number of well-known

methods, e.g. by conversion to an acyl halide or to an active ester such as the N-hydroxysuccinimide ester. The resultant activated carboxyl may then be easily converted to simple or multifuntional ester, amide, or thioester derivatives by reaction with alcohols, amines, or thiols respectively, alone or in the presence of condensation catalysts. For example, 4-carboxy-6-(N-decanoylamino)indole may be converted to its N-hydroxysuccinimide ester by reaction with one equivalent of dicyclohexylcarbodiimide and one equivalent of N-hydroxysuccinimide in acetonitrile/tetrahydrofuran/methylene chloride suspension. The product N-hydroxysuccinimide ester is purified and then reacted with 3-amino-1,2-propanediol in tetrahydrofuran to obtain 4-(N-2,3-dihydroxypropylcarboxamido)-6-(N-decanoylamino)indole.

The use of the methods of total synthesis as described in the literature cited above, with obvious adaptations, permits specific modifications of the parent structures in the diterpene, indolactam, diacyglycerol, and diaminobenzyl alcohol groups, by established techniques in the art of synthetic chemistry, to obtain modified parent structures emboying alterations at the hydroxymethyl or 1-hydroxyethyl groups and having anti-inflammatory activity.

To further illustrate the synthesis of the compounds of this invention, the modified indolactam, 1,2,4,5,6,8-hexahydro-5-methyl-2-(1-methylethyl)-3H-pyrrolo(4,3,2-gh)-1,4-benzodiazonin-3-one, may be prepared from N-BOC-4-nitrotryptophanol [Y. Endo et al., Tetrahedron, 42, 5905-5924 (1986)] by the application of several routes obvious to workers with ordinary skill in synthetic chemistry. For example, preparation of N-BOC-4-nitrodeoxytryptophanol may be accomplished by reduction of the phenylselenium derivative with triphenyltinhydride [D. Clive et al., Chemical Communications, 41-42 (1978)] or by reduction of the mesylate derivative with lithium triethylborohydride [R.W. Holder and M.G. Matturro, J. Org. Chem., 42, 2166-2168 (1977)] or by several other routes. From the deoxy derivative the synthesis could proceed in the manner described by Y. Endo et al., loc cit., for the hydroxy derivative. Specifically, the resulting substituted indolylvaline methyl ester is hydrolysed and the resulting acid is converted to the N--succimidyl ester. Upon cleavage of the BOC group under acidic conditions cyclization to the lactam occurs directly to provide all four stereoisomers, i.e., 2R,5R-, 2R,5S-, 2S,5S-, and 2S,5R-1,2,4,5,6,8-hexahydro-5-methyl-2-(1-methylethyl)-3H-pyrrolo (4,3,2-gh)-1,4-benzodiazonin-3-one. These stereoisomers may be obtained separately either by beginning the synthesis with optically active N-BOC-4-nitrotrytophanol or by separation from the mixture by chromatography on an enantioselective column packing [D. Armstrong, Analytical Chem., 59, 84-91A (1987)]. Similarly, the further modified indolactam, 1-(1-oxobutyl)-1,2,4,5,6,8-hexahydro-5-methyl-3H-pyrrolo(4,3,2-gh)-1,4-benzodiazonin-3-one, may be prepared. Specifically, hydrogenation of N-BOC-4-nitrodeoxytrytophanol over palladium on carbon will provide N(2')-BOC-4-aminodeoxytrytophanol. Alkylation of this compound with methyl bromoacetate affords N-[3-(N-BOC-2-aminopropyl)-4-indolyl]glycine methyl ester [Y. Endo, et al., Chem. Pharm. Bull, 30, 3457-3460 (1982)]. Acylation of this material with butanoyl chloride in the presence of potassium carbonate or pyridine will provide N-butanoyl-N-[3-(N-BOC-2-aminopropyl)-4-indolyl]glycine methyl ester. This latter material may be converted to 1-(1-oxobutyl)-1,2,4,5,6,8-hexahydro-5-methyl-3H-pyrrolo(4,3,2-gh)-1,4-benzodiazonin-3-one by application of the methods of Y. Endo, et al., loc cit. (1986). The enantiomers, 5R- and 5S-, may be obtained separately by beginning with optically active materials as described above or by separation at the final stage by chromatography also as described above.

This invention is illustrated further by the following examples.

## EXAMPLE 1

One gram of sodium metal was dissolved in 50 ml methanol, and 0.044 ml of this solution was placed in a test tube. Then 2.63 grams distilled 2-mercaptoethanol was dissolved in 50 ml acetonitrile, and 0.044 ml of this solution was added to the test tube. Then 20 mg 20-deoxy-20-chlorophorbol 12,13-dibutyrate were dissolved in 0.25 ml acetonitrile in a capped, nitrogen-flushed test tube. This latter solution was then rapidly treated with the methoxide/mercaptoethanol solution. An immediate precipitate formed. After 7 minutes, the reaction was freed of solvent and treated with 1 ml water and 1 drop acetic acid. This residue was partitioned between water and ethyl acetate, followed by drying of the organic phase over sodium sulfate. Silica gel preparative liquid chromatography using hexaneethyl acetate mixtures yielded 9.5 mg of 20-deoxy-20-(2-hydroxyethylthio)phorbol 12,13-dibutyrate, which could not be crystallized.

## EXAMPLE 2

Further preparative liquid chromatography of the reaction mixture from Example 1 using hexaneethyl acetate on siliza gel yielded 5.7 mg 20-deoxy-20-(2-hydroxyethylthio)phorbol 12-butyrate, the latter being the more polar compound. This compound could not be crystallized.

## EXAMPLE 3

To 0.1 gram 20-deoxy-20-chlorophorbol 12-myristate 13-acetate in 1 ml acetonitrile was added 0.5 ml of a solution of 246 mg 2-mercaptoethanol and 436 mg 2,4,6-collidine in 10 ml acetonitrile, followed by 50 mg diisopropylethylamine in 0.2 ml acetonitrile and 0.1 ml t-butyl methyl ether. Ten minutes later the reaction was treated with 0.107 mmoles sodium methoxide and 0.225 mmoles 2-mercaptoethanol in 0.25 ml methanol. Five minutes later the proportion of

sodium methoxide/mercaptoethanol was doubled. After 10 minutes the reaction was stopped by addition of 2 drops acetic acid and 0.5 ml water. The organics were extracted into ethyl acetate, washed once with water, and dried over sodium sulfate. After solvent removal, the crude residue was purified by preparative liquid chromatograhpy on silica gel using hexane/ethyl acetate 60/40. The product was 87 mg 20-deoxy-20-(2-hydroxyethylthio)phorbol 12-myristate 13-acetate in high purity. The compound did not crystallize.

EXAMPLE 4

Twenty-five mg of 20-deoxy-20-chlorophorbol 12-myristate 13-acetate was dissolved in 0.2 ml ethylene glycol and 0.2 ml acetonitrile. This solution was treated with 0.13 ml of a solution of 200 mg sodium metal in 20 ml ethylene glycol over a period of 40 minutes. The reaction was partitioned between water and ethyl acetate, and the separated organics were dried over sodium sulfate. After removal of the ethyl acetate, the crude 20-deoxy-20-chlorophorbol 12-myristate was dissolved in 0.8 ml acetonitrile and treated with 0.18 ml of a solution of 0.31 ml 2-mercaptoethanol, 0.5 ml acetonitrile, and 0.5 ml of 1% sodium in methanol. After 40 minutes, an additional 0.04 ml of 1% sodium in methanol was added. Ten minutes later the reaction was stopped with 1 drop of acetic acid. After removal of the solvents in a stream of nitrogen, the residue was partitioned between ethyl acetate and pH 8 potassium phosphate. The organics were dried over sodium sulfate and freed of solvent prior to preparative liquid chromatographic purification using silica gel and hexane/ethyl acetate 45/55. The product, 37 mg of 20-deoxy-20-(2-hydroxyethylthio)phorbol 12-myristate, could not be crystallized.

EXAMPLE 5

25 mg 20-deoxy-20-chlorophorbol 12-myristate 13-acetate was dissolved in 0.4 ml acetonitrile. To this was added 0.1 ml of a solution of 29.6 mg 2-(methylamino)ethanol in 1 ml acetonitrile. After 70 minutes and additional 0.1 ml of the same amine solution was added. After an additional 70 minutes, 0.2 ml more amine solution was added. After 6.6 hours of total reaction time, the reaction was diluted with 4 ml methylene chloride and subjected to preparative liquid chromatography on silica gel using methylene chloride/methanol 92/8 followed by re-purification on silica gel using methylene chloride/methanol 96/4. The product, 20-deoxy-20-[(2-hydroxyethyl)methylamino]phorbol 12-myristate 13-acetate, 14 mg, could not be crystallized.

EXAMPLE 6, 7, and 8

100 mg of phorbol 12,13-bis(2,4-difluorophenylacetate) were dissolved in 1.5 ml methylene chloride and the solution was set at 0°C. Then 26.2 mg diethylaminosulfur trifluoride in 0.5 ml methylene chloride were added dropwise during 1 minute. After 40 minutes, 0.2 ml more diethylaminosulfur trifluoride was added. After 10 more more minutes, the reaction was shaken with 2 ml pH 8 potassium phosphate buffer, after which the organics were separated and dried over sodium sulfate. The reaction was repeated twice more, and the combined reaction products were freed of solvent, taken up in 10 ml ethyl acetate, and sucked through a funnel containing a layer of silica at the bottom, a layer of sodium sulfate in the middle and sodium chloride at the top. After washing the funnel contents with 50 ml ethyl acetate the combined eluants were freed of solvent and repeatedly chromatographed on silica preparative liquid chromatography columns using hexane/ethyl acetate 85/15 solvent mixtures. The products were 20-deoxy-20-fluorophorbol 12,13-bis(2,4-difluorophenylacetate), 40 mg; 12-beta,13-bis-(2,4-difluorophenylacetoxy)-4,9-dihydroxy-1, 6(20),7-tigliatrien-3-one, 25 mg; and 12-beta,13-bis(2,4-difluorophenylacetoxy)-4,9-dihydroxy-7-fluoro-1,6(20)-tigliaden-3-one, 40 mg; none of which could be crystallized.

EXAMPLE 9

Five hundred-fifteen mg of 4-carbomethoxy-6-(N-decanoylamino)indole [prepared by the method of Wender et al., Proc. Natl. Acad. Sci. US.A., 83, 4214-4218 (1986)] was dissolved in 60 mL of tetrahydrofuran. This solution was treated with 3 mL of a 1N KOH solution in water and also with 5 mL of methanol. The mixture was heated at 80°C for 32 h. During this period another 2.5 mL of 1N KOH was added in two portions. After cooling the mixture was concentrated in vacuo. The mixture was then diluted with water and acidified with concentrated hydrochloric acid. The mixture was then extracted with methylene chloride. The organic layers were dried over sodium sulfate and concentrated to afford 300 mg of 4-carboxy-6-(N-decanoylamino) indole (60% yield). mp 248-50°C.

EXAMPLE 10

A suspension of 470 mg of 4-carboxy-6-(N-decanoylamino)indole and 427 mg of N-hydroxysuccinimide in 100 mL of acetonitrile, 10 mL of methylene chloride and 10 mL of tetrahydrofuran was prepared. This suspension was stirred

vigorously by a magnetic stirring bar as a solution of 540 mg of dicyclohexylcarbodiimide in 20 mL of acetonitrile was slowly added over a period of 1 h. The mixture was stirred vigorously for 72 h. It was then concentrated in vacuo and diluted with ethyl acetate. The resulting mixture was filtered to remove the copious precipitate. The filtrate was washed with water, dried over sodium sulfate and concentrated. Treatment of the resulting mixture with hexane/ethyl acetate 50:50 followed by filtration afforded 600 mg of 4-carboxy-6-(N-decanoylamino)indole N-succinimidyl ester, mp 154-5°C.

EXAMPLE 11

To a solution of 89 mg of 3-amino-1,2-propanediol in 20 mL of tetrahydrofuran was added 136 mg of 4-carboxy-6-(N-decanoylamino)indole N-succinimidyl ester. The solution was stirred for 48 h. After concentration in vacuo the mixture was purified by preparative liquid chromatography using silica gel and methylene chloride/methanol 92:8. The product, 135 mg of 4-(N-2,3-dihydroxypropylcarboxamido)-6-(N-decanoylamino)indole, was recrystallized from methanol-methylene chloride, mp 139-41°C.

EXAMPLE 12

To a solution of 113 mg of triethylamine in 20 mL of tetrahydrofuran was added, first, 86 mg of 2-aminoethanethiol hydrochloride and, then, 139 mg of 4-carboxy-6-(N-decanoylamino)indole N-succinimidyl ester. The solution was stirred for 48 h and then concentrated in vacuo. Purification by liquid chromatography using silica gel and methylene chloride/methanol 96:4 afforded two products. The earlier eluting product, 23 mg of 4-(N-2-mercaptoethylcarboxamido)-6-(N-decanoylamino)indole, decomposed at 200-5°C. The later eluting product, 22 mg of 4-(S-2-aminoethylthiolcarboxy)-6-(N-decanoylamino)indole, was recrystallized from methanol, mp 192-3°C.

EXAMPLE 13

To a solution of 145 mg of 4-carboxy-6-(N-decylamino)indole N-hydroxysuccinimidyl ester in 20 mL of tetrahydrofuran was added 103 mg or piperidinemethanol. The solution was heated at reflux for 5 days, at which time another 100 mg of piperidinemethanol was added and heating continued for another day. The solution was then concentrated in vacuo and purified by liquid chromatography using silica gel and methylene chloride/iso-propyl alcohol (93:7). In this manner 21 mg of 4-(2-hydroxymethylpiperidinocarbonyl)-6-(N-decanoylamino)indole wag obtained, M.P. 126-129°C.

EXAMPLE 14

One hundred milligrams of phorbol 12-myristate 13-acetate was dissolved in 1 ml tetrahydrofuran. To this solution was added 11.5 microliters of methyl isocyanate, followed immediately by 20 microliters of a 10% by weight solution of dibutyltin dilaurate in tetrahydrofuran. After three hours an additional 11.5 microliters of methyl isocyanate was added. Sixteen hours later 30 microliters of the reaction solution was applied to a silica gel TLC plate and developed with hexanes/ethyl acetate 46/54. The band at Rf 0.4 was scraped off and the product was eluted from the silica with acetone. Removal of the solvent in a stream of nitrogen gave 2.8 mg of phorbol 12-myristate 13-acetate 20-methylcarbamate as a glassy solid for spectroscopic analysis and bioassay.

EXAMPLE 15

Ninety-eight mg of 20-deoxy-20-chlorophorbol 12-myristate 13-acetate was dissolved in 0.5 ml tetrahydrofuran. To this was added 0.15 ml saturated aqueous ammonium chloride, 0.15 ml 37% formalin solution, and 50 mg zinc dust (less than 325 mesh). The reaction was capped and shaken vigorously for 24 hours. At the end of this time the reaction was partitioned between pH8 phosphate buffer and ethyl acetate. The ethyl acetate phase was reduced to a volume of 2 ml under a stream of nitrogen and 0.125 ml was applied to a silica gel TLC plate and developed with hexanes/ethyl acetate 46/54. The band at Rf 0.45 was scraped off and the product was eluted from the silica powder with acetone. Removal of the acetone yielded 3.0 mg of 20-homophorbol 12-myristate 13-acetate as a glassy solid for spectroscopid analysis and bioassay.

EXAMPLE 16

A stock solution of 300 pmoles of the standard inflammatory compound phorbol 12-myristate 13-acetate per 0.005 ml acetone was prepared. This solution was used to prepare four-fold dilutions of 20-deoxy-20-(2-hydroxyethylthio)phorbol 12-myristate 13-acetate, prepared as in Example 3, covering concentrations of the latter ranging from 4 to 64,000 pmoles per 0.005 ml. These solutions were used to demonstrate the anti-inflammatory activity of the latter compound by application of 0.005 ml to the insides of the right ears of mice, followed by the observation of ear inflammation/erythema during a 1-48 hour period. Inhibition of the phorbol 12-myristate 13-acetate induced inflammation was observed at the medium and higher concentrations of the inhibitor.

In a like manner, the anti-inflammatory activities of the following other compounds were demonstrated:

phorbol 12-myristate
phorbol 12,13-diacetate
20-deoxy-20-chlorophorbol 12-myristate 13-acetate
20-deoxy-20-(2-hydroxyethylthio)phorbol 12,13-dibutyrate
20-deoxy-20-(2-hydroxyethylthio)phorbol 12-myristate 13-acetate
20-deoxy-20-[(2-hydroxyethyl)methylamino]phorbol 12-myristate 13-acetate
20-deoxy-20-fluorophorbol 12,13-bis(2,4-difluorophenylacetate)
12-beta,13-bis(2,4-difluorophenylacetoxy)-4,9-dihydroxy-1,6(20),7-tigliatrien-3-one
12-beta,13-bis(2,4-difluorophenylacetoxy)-4,9-dihydroxy-7-fluoro-1,6(20)-tigliadien-3-one
20-deoxy-20-hydroxymethylphorbol 12-myristate 13 acetate
phorbol 12-myristate 13-acetate 20-methylcarbamate.

**Claims**

1. A non-toxic compound for use in therapy derived from a toxic phorboid parent compound of any of the diterpene-phorboid class, the indolactam-phorboid class, the polyacetate-phorboid class, and the bryostatin-phorboid class, said toxic phorboid parent compound binding reversibly or irreversibly to a diacylglycerol-type receptor and/or activating any form of protein kinase C and containing a hydroxymethyl or 1-hydroxyethyl group bound to a carbon atom, wherein (a) the hydroxymethyl or 1-hydroxyethyl group is replaced by a substituent group singly or doubly bound to said carbon atom, which results in loss of the toxic properties; or (b) the hydroxymethyl or 1-hydroxyethyl group is absent and a substituent group which results in loss of the toxic properties is singly or doubly bound to a carbon atom immediately adjacent the carbon atom to which the hydroxymethyl or 1-hydroxyethyl group is bound in the parent compound.

2. A compound according to claim 1 wherein the substituent group blocks the diacylglycerol activation of protein kinase C.

3. A compound according to claim 1 or claim 2 wherein the substituent group is represented by $S_o$-$E_o$; wherein $S_o$ is a saturated or unsaturated, straight or branched chain of atoms which separates $E_o$ from the remainder of the compound by a linear count of at least 2 but not more than 6 atoms and contains:

   1 to 6 heteroatoms selected from oxygen, nitrogen and sulfur;
   a phosphorus atom; or
   up to 12 halogen atoms;
   provided that the total number of atoms does not exceed 35; and
   $E_o$ comprises a saturated or singly or multiply unsaturated group containing up to 8 carbon atoms and optionally 1 to 12 halogen atoms, 1 to 4 heteroatoms selected independently from oxygen, nitrogen, sulfur and one phosphorus atom; or wherein
   $S_o$-$E_o$ taken together may be a hydrogen, halogen, or ketonic oxygen atom or a hydroxy, amine, or thiol group singly or doubly bound to the carbon atom in replacement of the hydroxymethyl or 1-hydroxyethyl group.

4. A compound according to any one of claims 1, 2 and 3 in the form of an individual isomer, an isomer mixture, a racemate or optical antipode, or a pharmaceutically acceptable salt thereof.

5. A compound (e.g. for use in therapy) selected from:

   12,20-dideoxy-20-chlorophorbol 13-angelate;
   20-deoxy-20-bromophorbol 12-myristate 13-acetate;
   20-deoxy-20-iodophorbol 12-myristate 13-acetate;
   20-deoxy-20-chlorophorbol 12,13-bis(2,4-difluorophenyl acetate);
   20-deoxy-20-fluorophorbol 12,13-bis(2,4-difluorophenyl acetate);
   12-beta,13-bis(2,4-difluozophenylacetoxy)-4,9-dihydroxy-1,6(20),7-tigliatrien-3-one;
   12-beta,13-bis(2,4-diflurophenylacetoxy)-4,9-dihydroxy-7-fluoro-1,6(20)-tigliadien-3-one;
   20-deoxy-20-cyanophorbol 12-myristate 13-acetate;
   20-deoxy-20-(2-hydroxyethylthio)phorbol 12-butyrate;

20-deoxy-20-(2-hydroxyethylthio)phorbol 12,13-dibutyrate;
20-deoxy-20-(2-hydroxyethylthio)phorbol 12-myristate 13-acetate;
20-deoxy-20-[(2-hydroxyethyl)methylamino]phorbol 12-myristate 13-acetate;
20-deoxy-20-(3-hydroxypropylthio)phorbol 12,13-bis(2,4-difluorophenylacetate);
20-deoxy-20-aminophorbol 12-myristate 13-acetate and pharmaceutically acceptable salts thereof;
20-deoxy-20-N-methylaminophorbol 12-myristate 13-acetate and pharmaceutically acceptable salts thereof;
20-deoxy-20-hydroxymethylphorbol 12-myristate 13-acetate;
20-deoxy-20-(1-hydroxyethyl)phorbol 12-myristate 13-acetate;
20-deoxy-20-(2-hydroxyethyl)phorbol 12-myristate 13-acetate;
20-deoxy-20-(2-hydroxyethyl)phorbol 12,13-bis(2,4-diflurophenylacetate);
12,20-dideoxy-20-(2-hydroxyethylthio)phorbol 13-angelate;
phorbol 12-myristate 13-acetate 20-methylcarbamate;
phorbol 12-myristate 13-acetate 20-ethylcarbamate;
phorbol 12-myristate 13-acetate 20-n-propylcarbamate;
phorbol 12-myristate 13-acetate 20-n-butylcarbamate;
phorbol 12-myristate 13-acetate 20-(2-hydroxymethylphenyl) ether;
20-deoxy-20-(1-tetrazolyl)phorbol 12-myristate 13-acetate;
1-(1-oxobutyl)-1,2,4,5,6,8-hexahydro-5-methyl-3H-pyrrolo(4,3,2-gh)-1,4-benzodiazonin-3-one;
1,2,4,5,6,8-hexahydro-5-methyl-2-(1-methylethyl)-3H-pyrrolo(4,3,2-gh)-1,4-benzodiazonin-3-one;
teleocidin 20-methylcarbamate;
teleocidin 20-n-butylcarbamate;
lyngbyatoxin A 20-methylcarbamate;
lyngbyatoxin A 24-n-butylcarbamate;
aplysiatoxin 20-methyl ether 30-methylcarbamate;
aplysiatoxin 20-acetate 30-methylcarbamate;
aplysiatoxin 20,30-bis(methylcarbamate);
the 26-methylcarbamate of bryostatin 1;
the 26-n-butylcarbamate of bryostatin 1.

6. A compound acording to any one of claims 1 to 5 wherein the therapy comprises the treatment of any of: inflammatory conditions, cancer, leukemia, asthma, hypertension, parasitic infection, psoriasis, ulcers, arthritis, autoimmune disease, amoebic disease, HTLV-III/LAV viral replication and Alzheimer's disease.

7. A compound according to any one of claims 1 to 5, wherein the therapy is anti-nociceptive or comprises any of insulin secretion, or insulinomimetic activity, stimulation of production of lymphokines (e.g. interferon) or interleukins, tumor necrosis factor production, immune system stimulation and/or reconstitution, acceleration of wound healing and improvement in central nervous system functions, e.g. memory and learning.

8. An antagonist of protein kinase C, a non-toxic agonist of protein kinase C, an antagonist for toxic phorboids, or a non-toxic phorboid-type agonist, comprising a compound as defined in any one of claims 1 to 5, excluding:

a) diterpene compounds having carboxyl, carboxaldehyde, carboxaldehyde-2,4,-dinitrophenyl-hydrazone, cyano, alkanoyloxymethylene, alkyoxymethylene chloromethyl or methyl in place of the hydroxymethyl group;
b) indole compounds having chloromethyl, methyl, alkanoyloxymethylene or alkoxymethylene in place of the hydroxymethyl group.

9. Use, for the manufacture of a medicament for the treatment of conditions in which protein kinase C is pathologically or therapeutically involved, of a non-toxic compound derived from a toxic phorboid parent compound which binds reversibly or irreversibly to a diacylglycerol-type receptor and/or activates any form of protein kinase C and contains a hydroxymethyl or 1-hydroxyethyl group bound to a carbon atom, wherein (a) the hydroxymethyl or 1-hydroxyethyl group is replaced by a substituent group, singly or doubly bound to said carbon atom, which results in loss of the toxic properties; or (b) the hydroxymethyl or 1-hydroxyethyl group is absent and a substituent group which results in loss of the toxic properties is singly or doubly bound to a carbon atom immediately adjacent the carbon atom to which the hydroxymethyl or 1-hydroxyethyl group is bound in the parent compound.

10. Use of a compound as defined in any one of claims 1 to 5; or claim 9, for the manufacture of a medicament for the treatment of any of: inflammatory conditions, cancer, leukemia, asthma, hypertension, parasitic infection, psoriasis, ulcers, arthritis, auto-immune disease, amoebic disease, HTLV-III/LAV viral replication and Alzheimer's disease.

11. Use of a compound as defined in any one of claims 1 to 5; or claim 9 for the manufacture of a medicament for use in therapy which is anti-nociceptive or comprises any of insulin secretion, insulinomimetic activity, or the stimulation of the production of lymphokines (e.g. interferon) or interleukins, tumor necrosis factor production, immune system stimulation and/or reconstitution, acceleration of wound healing and improvement in central nervous system functions, e.g. memory and learning.

12. A pharmaceutical composition containing a compound as defined in any one of claims 1 to 5 or claim 9 and a pharmaceutically acceptable carrier.

13. A method of producing a substance for use in therapy comprising a non-toxic phorboid antagonist or a non-inflammatory agonist ligand selective for one or more phorboid receptors or protein kinase C subtypes, comprising replacing a hydroxymethyl or 1-hydroxyethyl group bound to a carbon atom in a toxic parent phorboid compound which parent phorboid compound binds reversibly or irreversibly to a diacylglycerol-type receptor and/or activates any form of protein kinase C, with a group which is singly or doubly bound to the carbon atom to which the hydroxymethyl or 1-hydroxyethyl group is bound in the toxic phorboid compound or to an immediately adjacent carbon atom, and is represented by $S_o$-$E_o$; wherein $S_o$ is a saturated or unsaturated, straight or branched chain of atoms which separates $E_o$ from the remainder of the compound by a linear count of at least 2 but not more than 6 atoms and contains:

 1 to 6 heteroatoms selected from oxygen, nitrogen and sulfur;
 a phosphorus atom; or
 up to 12 halogen atoms;
 provided that the total number of atoms does not exceed 35; and
 $E_o$ comprises a saturated or singly or multiply unsaturated group containing up to 8 carbon atoms and optionally 1 to 12 halogen atoms, 1 to 4 heteroatoms selected independently from oxygen, nitrogen, sulfur and one phosphorus atom; or wherein
 $S_o$-$E_o$ taken together may be a hydrogen, halogen, or ketonic oxygen atom or a hydroxy, amine, or thiol group singly or doubly bound to the carbon atom in replacement of the hydroxymethyl or 1-hydroxyethyl group.

14. Use of a compound as defined in any one of claims 1 to 5 or claim 9 in diagnosis in vitro, e.g. in an assay for protein kinase C.

15. Use in vitro of a compound as defined in any one of claims 1 to 5; or claim 9 as a phorboid receptor subtype- and/or protein kinase C subtype-selective ligand.

16. Use of a compound as defined in any one of claims 1 to 5; or claim 9 as a pharmacological tool for in vitro research.

17. Use of a compound as defined in any one of claims 1 to 5; or claim 9 (excluding resiniferatoxin and tinyatoxin) as a pharmacological tool for in vivo research excluding methods of treatment of the human or animal body by therapy and diagnostic methods practised on the human or animal body.

18. Use of a compound as defined in any one of claims 1 to 5; or claim 9 as an intermediate in the production of another drug.

19. A compound of the formula:

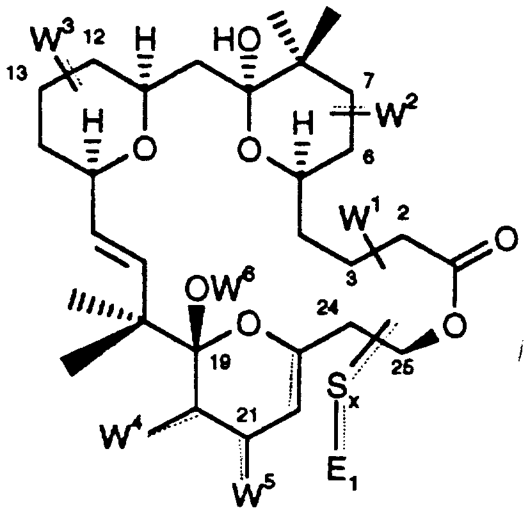

in the form of an individual isomer, an isomer mixture, a racemate or optical antipode, or a pharmaceutically acceptable salt thereof,
wherein $W^1$ and $W^2$ are selected from hydrogen, halogen, hydroxy, $C_{1-5}$ alkoxy, $C_{1-5}$ alkanoyloxy and cyano; $W^3$-$W^5$ each may be hydrogen or a straight chain or branched chain, cyclic or acyclic, saturated, unsaturated, and/or aromatic carbon- and/or heteroatom-containing group, containing not more than 30 carbon atoms, not more than 24 halogen atoms, and not more than 8 heteroatoms selected from oxygen, nitrogen, and sulfur, and $W^4$ and $W^5$ taken together may form an additional carbocyclic or heterocyclic ring; and $W^6$ is hydrogen;
wherein $S_xE_1$ comprises a substituent group bound to carbon 25, wherein $S_x$ is a saturated or unsaturated, straight or branched chain of atoms which separates $P_6$ and $E_1$ by a linear count of not more than 6 atoms and may contain: one to six heteroatoms selected from oxygen, nitrogen and sulfur; a phosphorus atom; or up to 12 halogen atoms; provided that the total number of atoms does not exceed 35; and
wherein $E_1$ comprises a saturated or singly or multiply unsaturated group containing up to 8 carbon atoms and optionally containing 1 to 12 halogen atoms, 1 to 4 heteroatoms selected independently from oxygen, nitrogen, sulfur and one phosphorus atom, or wherein
$S_xE_1$ taken together may be a hydrogen, halogen, or ketonic oxygen atom or a hydroxy, amine, or thiol group singly or doubly bound to a carbon atom of a corresponding parent compound in replacement of a hydroxymethyl or 1-hydroxyethyl group of said parent compound, provided that $S_xE_1$ is not hydroxymethyl, 1-hydroxyethyl, or acylated 1-hydroxyethyl.

**Patentansprüche**

1. Eine nicht-toxische Verbindung zum Gebrauch in der Therapie, abgeleitet aus einer toxischen phorboiden Ausgangsverbindung einer beliebigen der folgenden Klassen: der diterpen-phorboiden Klasse, der indolaktam-phorboiden Klasse, der polyacetat-phorboiden Klasse oder der briostatin-phorboiden Klasse, wobei die genannte toxische phorboide Ausgangsverbindung reversibel oder irreversibel an einen Rezeptor des Diacylglycerin-Typs gebunden ist und/oder eine beliebige Form der Protein-Kinase C aktiviert und eine Hydroxymethyl- oder 1-Hydroxyethylgruppe enthält, die an ein Kohlenstoffatom gebunden ist, worin

   (a) die Hydroxymethyl- oder 1-Hydroxyethylgruppe ersetzt ist durch eine Substituentengruppe, die einfach oder doppelt an das besagte Kohlenstoffatom gebunden ist, was zu dem Verlust der toxischen Eigenschaften führt; oder
   (b) die Hydroxymethyl- oder 1-Hydroxyethylgruppe fehlt und eine Substituentengruppe, die zu einem Verlust der toxischen Eigenschaften führt, einfach oder doppelt an das Kohlenstoffatom gebunden ist unmittelbar benachbart dem Kohlenstoffatom, an das die Hydroxymethyl- oder 1-Hydroxyethylgruppe in der Ausgangsverbindung gebunden ist.

2. Eine Verbindung nach Anspruch 1, in der die Substituentengruppe die Diacylglycerinaktivierung der Protein-Kinase C blockiert.

3. Eine Verbindung nach einem der Ansprüche 1 oder 2, worin die Substituentengruppe gebildet ist durch $S_0$-$E_0$, worin $S_0$ eine gesättigte oder ungesättigte unverzweigte oder verzweigte Kette von Atomen ist, die $E_0$ vom Rest der Verbindung durch eine lineare Anzahl von wenigstens 2, aber nicht mehr als 6 Atomen trennt, und die enthält:

1 bis 6 Heteroatome, ausgewählt aus Sauerstoff, Stickstoff und Schwefel;
ein Phosphoratom;
oder bis zu 12 Halogenatome;
ausgebildet derart, daß die Gesamtzahl von Atomen 35 nicht überschreitet und
$E_0$ eine gesättigte oder einfach oder mehrfach ungesättigte Gruppe umfaßt, die bis zu 8 Kohlenstoffatome und optional 1 bis 12 Halogenatome, 1 bis 4 Heteroatome, unabhängig voneinander ausgewählt aus Sauerstoff, Stickstoff, Schwefel, und ein Phosphoratom enthält, oder worin
$S_0$-$E_0$ zusammengenommen ein Wasserstoff-, Halogen- oder ketonisches Sauerstoffatom oder eine Hydroxy-, Amino-, oder Thiolgruppe, einfach oder doppelt an das Kohlenstoffatom in Ersetzung der Hydroxymethyl- oder 1-Hydroxyethylgruppe gebunden, sein kann.

4. Eine Verbindung nach einem der Ansprüche 1, 2 oder 3 in der Form eines einzelnen Isomers, einer Isomermischung, eines Racemats oder optischer Antipode oder eines pharmazeutisch verwendbaren Salzes hiervon.

5. Eine Verbindung (z.B. zum Gebrauch in der Therapie), ausgewählt aus:

12,20-dideoxy-20-chlorophorbol 13-angelate;
20-deoxy-20-bromophorbol 12-myristate 13-acetate;
20-deoxy-20-iodophorbol 12-myristate 13-acetate;
20-deoxy-20-chlorophorbol 12,13-bis(2,4-difluorophenyl acetate);
20-deoxy-20-fluorophorbol 12,13-bis(2,4-difluorophenyl acetate);
12-beta,13-bis(2,4-difluorophenylacetoxy)-4,9-dihydroxy-1,6(20),7-tigliatrien-3-one;
12-beta,13-bis(2,4-diflurophenylacetoxy)-4,9-dihydroxy-7-fluoro-1,6(20)-tigliadien-3-one;
20-deoxy-20-cyanophorbol 12-myristate 13-acetate;
20-deoxy-20-(2-hydroxyethylthio)phorbol 12-butyrate;
20-deoxy-20-(2-hydroxyethylthio)phorbol 12,13-dibutyrate;
20-deoxy-20-(2-hydroxyethylthio)phorbol 12-myristate 13-acetate;
20-deoxy-20-[(2-hydroxyethyl)methylamino]phorbol 12-myristate 13-acetate;
20-deoxy-20-(3-hydroxypropylthio)phorbol 12,13-bis(2,4-difluorophenylacetate);
20-deoxy-20-aminophorbol 12-myristate 13-acetate und pharmazeutisch verwendbare Salze hiervon;
20-deoxy-20-N-methylaminophorbol 12-myristate 13-acetate und pharmazeutisch verwendbare Salze hiervon;
20-deoxy-20-hydroxymethylphorbol 12-myristate 13-acetate;
20-deoxy-20-(1-hydroxyethyl)phorbol 12-myristate 13-acetate;
20-deoxy-20-(2-hydroxyethyl)phorbol 12-myristate 13-acetate;
20-deoxy-20-(2-hydroxyethyl)phorbol 12,13-bis(2,4-diflurophenylacetate);
12,20-dideoxy-20-(2-hydroxyethylthio)phorbol 13-angelate;
phorbol 12-myristate 13-acetate 20-methylcarbamate;
phorbol 12-myristate 13-acetate 20-ethylcarbamate;
phorbol 12-myristate 13-acetate 20-n-propylcarbamate;
phorbol 12-myristate 13-acetate 20-n-butylcarbamate;
phorbol 12-myristate 13-acetate 20-(2-hydroxymethylphenyl) ether;
20-deoxy-20-(1-tetrazolyl)phorbol 12-myristate 13-acetate;
1-(1-oxobutyl)-1,2,4,5,6,8-hexahydro-5-methyl-3H-pyrrolo(4,3,2-gh)-1,4-benzodiazonin-3-one;
1,2,4,5,6,8-hexahydro-5-methyl-2-(1-methylethyl)-3H-pyrrolo(4,3,2-gh)-1,4-benzodiazonin-3-one;
teleocidin 20-methylcarbamate;
teleocidin 20-n-butylcarbamate;
lyngbyatoxin A 20-methylcarbamate;
lyngbyatoxin A 24-n-butylcarbamate;
aplysiatoxin 20-methyl ether 30-methylcarbamate;
aplysiatoxin 20-acetate 30-methylcarbamate;
aplysiatoxin 20,30-bis(methylcarbamate);
the 26-methylcarbamate of bryostatin 1;
the 26-n-butylcarbamate of bryostatin 1.

6. Eine Verbindung nach einem der Ansprüche 1 bis 5, worin die Therapie die Behandlung von beliebigen der folgenden Krankheiten umfaßt: Entzündungen, Krebs, Leukämie, Asthma, Bluthochdruck, parasitische Infektion, Psoriasis, Geschwüre, Arthritis, Erkrankung des Immunsystems, durch Amöben hervorgerufene Erkrankung, HTLV-III/LAV Virenvermehrung und Alzheimersche Krankheit.

7. Eine Verbindung nach einem der Ansprüche 1 bis 5, wobei die Therapie eine anti-nozizeptive ist oder beliebige der folgenden Bestandteile umfaßt: Insulinsekretion oder insulinomimetische Aktivität, Stimulierung der Produktion von Lymphokinen (z.B. Interferon) oder Interleukinen, Produktion eines Tumor abtötenden Wirkstoffes, Immunsystemstimulierung und/oder Wiederherstellung, Beschleunigung der Wundheilung und Verbesserung von Funktionen des zentralen Nervensystems, z. B. des Gedächtnisses und der Lernfähigkeit.

8. Ein Gegenmittel der Protein-Kinase C, ein nicht-toxisches Fördermittel der Protein-Kinase C, ein Gegenmittel für toxische Phorboide, oder ein nicht-toxisches phorboid-artiges Fördermittel, das eine Verbindung wie beschrieben in einem der Ansprüche 1 bis 5 umfaßt, wobei ausgeschlossen ist:

   a) Diterpenverbindungen mit einer Karboxyl-, Karboxaldehyd-, Karboxaldehyd-2,4,-Dinitrophenyl-Hydrazon-, Cyan-, Alkanoyloxymethylen-, Alkyoxymethylenchlormethyl- oder Methyl-Gruppe anstelle der Hydroxymethylgruppe;

   b) Indolverbindungen mit einer Chlormethyl-, Methyl-, Alkanoyloxymethylen- oder Alkoxymethylengruppe anstelle der Hydroxymethylgruppe.

9. Verwendung für die Herstellung eines Medikamentes für die Behandlung von Zuständen, in die die Protein-Kinase C pathologisch oder therapeutisch eingebunden ist, einer nicht-toxischen Verbindung, erhalten aus einer toxischen phorboiden Ausgangsverbindung, die reversibel oder irreversibel an einen Rezeptor des Diacylglycerintyps bindet und/oder eine der folgenden Formen der Protein-Kinase C aktiviert und eine an ein Kohlenstoffatom gebundene Hydroxymethyl- oder 1-Hydroxyethylgruppe enthält, worin

   (a) die Hydroxymethyl- oder 1-Hydroxyethylgruppe ersetzt ist durch eine Substituentengruppe, die einfach oder doppelt an das besagte Kohlenstoffatom gebunden ist, was zu dem Verlust der toxischen Eigenschaften führt; oder
   (b) die Hydroxymethyl- oder 1-Hydroxyethylgruppe fehlt und eine Substituentengruppe, die zu einem Verlust der toxischen Eigenschaften führt, einfach oder doppelt an das Kohlenstoffatom gebunden ist unmittelbar benachbart dem Kohlenstoffatom, an das die Hydroxymethyl- oder 1-Hydroxyethylgruppe in der Ausgangsverbindung gebunden ist.

10. Verwendung einer Verbindung wie beschrieben in einem der Ansprüche 1 bis 5 oder 9 für die Herstellung eines Medikamentes für die Behandlung von: Entzündungen, Krebs, Leukämie, Asthma, Bluthochdruck, parasitischer Infektion, Psoriasis, Geschwüre, Arthritis, Erkrankung des Auto-Immunsystems, durch Amöben hervorgerufene Krankheit, HTLV-III/LAV Virenvermehrung und Alzheimerischer Krankheit.

11. Verwendung einer Verbindung wie beschrieben in einem der Ansprüche 1 bis 5 oder 9 für die Herstellung eines Medikamentes zum Gebrauch in der Therapie, die anti-nozizeptiv ist oder irgendwelche der folgenden Schritte umfaßt: Insulinsekretion, insulinomimetische Aktivität oder die Stimulierung der Produktion von Lymphokinen (z.B. Interferon) oder Interleukinen, Produktion von Tumor tötenden Wirkstoffen, Immunsystemanregung und/oder Wiederherstellung, Beschleunigung der Wundheilung und Verbesserung der Funktionen des zentralen Nervensystems, z.B. des Gedächtnisses und der Lernfunktion.

12. Eine pharmazeutische Zusammensetzung, die eine Verbindung wie beschrieben in einem der Ansprüche 1 bis 5 oder 9 enthält, und ein pharmazeutisch verwendbarer Träger.

13. Ein Verfahren zur Herstellung einer Substanz für den Gebrauch in der Therapie, umfassend einen nicht-toxischen phorboiden Antagonisten oder einen nicht-entzündungsfördernden Liganden, der selektiv ist für einen oder mehrere phorboide Rezeptoren oder Unterarten der Protein-Kinase C, umfassend das Ersetzen einer an ein Kohlenstoffatom gebundenen Hydroxymethyl- oder 1-Hydroxyethylgruppe in einer toxischen ausgangsphorboiden Verbindung, wobei diese ausgangsphorboide Verbindung reversibel oder irreversibel an einen Rezeptor des Diacylglycerin-Typs bindet und/oder eine Form der Protein-Kinase C aktiviert, mit einer Gruppe, die einfach oder doppelt an das Kohlenstoffatom, an dem die Hydroxymethyl- oder 1-Hydroxyethylgruppe in der toxischen phorboiden Verbindung gebunden ist, oder an ein unmittelbar benachbartes Kohlenstoffatom gebunden ist, und repräsentiert

ist durch $S_0$-$E_0$, worin $S_0$ eine gesättigte oder ungesättigte geradlinige oder verzweigte Kette von Atomen ist, die $E_0$ vom Rest der Verbindung trennt durch eine lineare Anzahl von wenigstens 2, aber nicht mehr als 6 Atomen, und die enthält:

1 bis 6 Heteroatome, ausgewählt aus Sauerstoff, Stickstoff und Schwefel;
ein Phosphoratom; oder bis zu 12 Halogenatome;
ausgebildet derart, daß die Gesamtzahl von Atomen 35 nicht überschreitet, und
$E_0$ eine gesättigte oder einfach oder mehrfach ungesättigte Gruppe umfaßt, die bis zu 8 Kohlenstoffatome und optional 1 bis 12 Halogenatome, 1 bis 4 Heteroatome, unabhängig voneinander ausgewählt aus Sauerstoff, Stickstoff, Schwefel, und ein Phosphoratom enthält, oder worin
$S_0$-$E_0$ zusammengenommen ein Wasserstoff-, Halogen- oder ketonisches Sauerstoffatom oder eine Hydroxy-, Amino-, oder Thiolgruppe, einfach oder doppelt an das Kohlenstoffatom in Ersetzung der Hydroxymethyl- oder 1-Hydroxyethylgruppe gebunden, sein kann.

**14.** Verwendung einer Verbindung wie beschrieben in einem der Ansprüche 1 bis 5 oder 9 zur In-vitro-Diagnose, z.B. in einem Assay für die Protein-Kinase C.

**15.** In-vitro-Verwendung einer Verbindung wie beschrieben in einem der Ansprüche 1 bis 5 oder 9 als ein phorboider Rezeptor-Untertyp und/oder ein nach Unterklassen der Protein-Kinase C selektiver Ligand.

**16.** Verwendung einer Verbindung wie beschrieben in einem der Ansprüche 1 bis 5 oder 9 als ein pharmakologisches Werkzeug für In-vitro-Forschung.

**17.** Verwendung einer Verbindung wie beschrieben in einem der Ansprüche 1 bis 5 oder 9 (unter Ausschluß von Resiniferatoxin und Tinyatoxin) als ein pharmakologisches Werkzeug für In-vivo-Forschung unter Ausschluß von Verfahren der Behandlung von menschlichen oder tierischen Körpern durch Therapie und diagnostische Methoden, die praktiziert werden an menschlichen oder tierischen Körpern.

**18.** Verwendung einer Verbindung wie beschrieben in einem der Ansprüche 1 bis 5 oder 9 als eine Zwischenverbindung in der Herstellung eines anderen Medikaments.

**19.** Eine Verbindung der Formel:

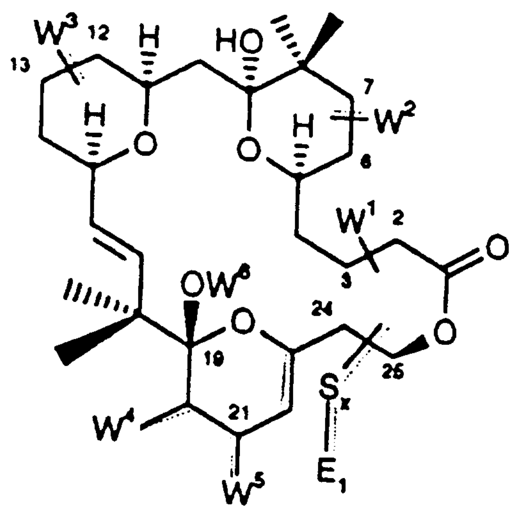

in der Form eines einzelnen Isomers, einer Isomermischung, eines Racemats oder einer optischen Antipode oder eines pharmazeutisch verwendbaren Salzes hiervon, worin $W^1$ und $W^2$ ausgewählt sind aus Wasserstoff, Halogen, einer Hydroxygruppe, einem $C_{1-5}$ Alkoxyd, einem $C_{1-5}$ Alkanoyloxyd und Cyan; $W^3$-$W^5$ können jeweils Wasserstoff oder eine geradlinige oder verzweigte Kette, zyklisch oder azyklisch, gesättigt oder ungesättigt und/oder eine aromatische Kohlenstoff- oder Heteroatomenthaltende Gruppe sein, die nicht mehr als 30 Kohlenstoffatome beinhaltet, nicht mehr als 24 Halogenatome und nicht mehr als 8 Heteroatome, ausgewählt aus Sauerstoff, Stickstoff und Schwefel, und wobei $W^4$ und $W^5$ zusammengenommen einen ergänzenden karbozyklischen oder heterozyklischen Ring bilden können und wobei $W^6$ Wasserstoff ist;

worin $S_xE_1$ eine Substituentengruppe umfaßt, die an den Kohlenstoff 25 gebunden ist, worin $S_x$ eine gesättigte oder ungesättigte, geradlinige oder verzweigte Kette von Atomen ist, die $P_6$ und $E_1$ separiert durch eine lineare Anzahl von nicht mehr als 6 Atomen und dabei enthalten kann: 1 bis 6 Heteroatome, ausgewählt aus Sauerstoff, Stickstoff und Schwefel, ein Phosphoratom oder bis zu 12 Halogenatome, derart vorgesehen, daß die Gesamtzahl von Atomen nicht den Wert von 35 überschreitet, und worin $E_1$ eine gesättigte oder einfach oder doppelt ungesättigte Gruppe umfaßt, die bis zu 8 Kohlenstoffatome und optional 1 bis 12 Halogenatome, 1 bis 4 Heteroatome, unabhängig ausgewählt aus Sauerstoff, Stickstoff, Schwefel, und ein Phosphoratom enthält, oder worin es $S_xE_1$ zusammengenommen ein Wasserstoff oder ein Halogen, oder ein ketonisches Sauerstoffatom sein kann oder eine Hydroxy-, Amino- oder Thiolgruppe, einzeln oder doppelt gebunden an ein Kohlenstoffatom einer korrespondierenden Ursprungsverbindung in Ersetzung einer Hydroxymethyl- oder 1-Hydroxyethylgruppe der besagten Ursprungsverbindung, derart ausgebildet, daß $S_xE_1$ nicht eine Hydroxymethyl-, 1-Hydroxyethyl, oder acylierte 1-Hydroxyethylgruppe ist.

**Revendications**

1. Composé non toxique à utiliser en thérapie, dérivé d'un composé phorboïde mère toxique choisi parmi l'une quelconque des classes comprenant la classe des diterpène-phorboïdes, la classe des indolactame-phorboïdes, la classe des polyacétate-phorboïdes et la classe des bryostatine-phorboïdes, ledit composé phorboïde mère toxique se liant de manière réversible ou irréversible à un récepteur du type du diacylglycérol et/ou activant n'importe quelle forme de la protéine-kinase C et contenant un groupe hydroxyméthyle ou un groupe 1-hydroxyéthyle lié à un atome de carbone, dans lequel (a) le groupe hydroxyméthyle ou le groupe 1-hydroxyéthyle a été remplacé par un groupe substituant lié de manière simple ou double audit atome de carbone, le remplacement donnant lieu à la perte des propriétés toxiques; ou (b) le groupe hydroxyméthyle ou le groupe 1-hydroxyéthyle est absent et un groupe substituant, qui donne lieu à la perte des propriétés toxiques, est lié de manière simple ou double à un atome de carbone immédiatement adjacent à l'atome de carbone auquel est lié le groupe hydroxyméthyle ou le groupe 1-hydroxyéthyle dans le composé mère.

2. Composé selon la revendication 1, dans lequel le groupe substituant bloque l'activation de la protéinase-kinase C par le diacylglycérol.

3. Composé selon la revendication 1 ou 2, dans lequel le groupe substituant est représenté par $S_0$-$E_0$ où $S_0$ représente une chaîne d'atomes saturée ou insaturée, droite ou ramifiée, qui sépare $E_0$ du reste du composé d'un nombre linéaire d'au moins 2 atomes, mais qui n'est pas supérieur à 6 atomes, et contient:

   de 1 à 6 hétéroatomes choisis parmi un atome d'oxygène, un atome d'azote et un atome de soufre;
   un atome de phosphore; ou
   jusqu'à 12 atomes d'halogène;
   avec cette réserve que le nombre total d'atomes ne dépasse pas 35; et
   $E_0$ comprend un groupe saturé ou à insaturation simple ou multiple contenant jusqu'à 8 atomes de carbone et, le cas échéant, de 1 à 12 atomes d'halogène, de 1 à 4 hétéroatomes choisis, indépendamment l'un de l'autre, parmi un atome d'oxygène, un atome d'azote, un atome de soufre, ainsi qu'un atome de phosphore; ou dans lequel
   $S_0$-$E_0$ pris ensemble peuvent représenter un atome d'hydrogène, un atome d'halogène ou un atome d'oxygène cétonique ou encore un groupe hydroxyle, un groupe amine ou un groupe thiol lié de manière simple ou double à l'atome de carbone, en remplacement du groupe hydroxyméthyle ou du groupe 1-hydroxyéthyle.

4. Composé selon l'une quelconque des revendications 1, 2 et 3, sous la forme d'un isomère individuel, d'un mélange d'isomères, d'un produit racémique ou d'un antipode optique, ou encore un de ses sels pharmaceutiquement acceptables.

5. Composé (par exemple à utiliser en thérapie) choisi parmi:

   le 13-angélate de 12,20-didésoxy-20-chlorophorbol;
   le 12-myristate 13-acétate de 20-désoxy-20-bromophorbol;
   le 12-myristate 13-acétate de 20-désoxy-20-iodophorbol;
   le 12,13-bis(2,4-difluorophénylacétate) de 20-désoxy-20-chlorophorbol;
   le 12,13-bis(2,4-difluorophénylacétate) de 20-désoxy-20-fluorophorbol;
   la 12-bêta,13-bis(2,4-difluorophénylacétoxy)-4,9-dihydroxy-1,6(20),7-tigliatrién-3-one;
   la 12-bêta,13-bis(2,4-difluorophénylacétoxy)-4,9-dihydroxy-7-fluoro-1,6(20)-tigliadién-3-one;

le 12-myristate 13-acétate de 20-désoxy-20-cyanophorbol;
le 12-butyrate de 20-désoxy-20-(2-hydroxyéthylthio)phorbol;
le 12,13-dibutyrate de 20-désoxy-20-(2-hydroxyéthylthio)phorbol;
le 12-myristate 13-acétate de 20-désoxy-20-(2-hydroxyéthylthio)phorbol;
le 12-myristate 13-acétate de 20-désoxy-20-[(2-hydroxyéthyl)méthylamino]phorbol;
le 12,13-bis(2,4-difluorophénylacétate) de 20-désoxy-20-(3-hydroxypropylthio)phorbol;
le 12-myristate 13-acétate de 20-désoxy-20-aminophorbol et ses sels pharmaceutiquement acceptables;
le 12-myristate 13-acétate de 20-désoxy-20-N-méthylaminophorbol et ses sels pharmaceutiquement acceptables;
le 12-myristate 13-acétate de 20-désoxy-20-hydroxyméthylphorbol;
le 12-myristate 13-acétate de 20-désoxy-20-(1-hydroxyéthyl)phorbol;
le 12-myristate 13-acétate de 20-désoxy-20-(2-hydroxyéthyl)phorbol;
le 12,13-bis(2,4-difluorophénylacétate) de 20-désoxy-20-(2-hydroxyéthyl)phorbol;
le 13-angélate de 12,20-didésoxy-20-(2-hydroxyéthylthio)phorbol;
le 12-myristate 13-acétate 20-méthylcarbamate de phorbol;
le 12-myristate 13-acétate 20-éthylcarbamate de phorbol;
le 12-myristate 13-acétate 20-n-propylcarbamate de phorbol;
le 12-myristate 13-acétate 20-n-butylcarbamate de phorbol;
le 12-myristate 13-acétate 20-(2-hydroxyméthylphényl)éther de phorbol;
le 12-myristate 13-acétate de 20-désoxy-20-(1-tétrazolyl) phorbol;
la 1-(1-oxobutyl)-1,2,4,5,6,8-hexahydro-5-méthyl-3H-pyrrolo(4,3,2-gh)-1,4-benzodiazonin-3-one;
la 1,2,4,5,6,8-hexahydro-5-méthyl-2-(1-méthyléthyl)-3H-pyrrolo(4,3,2-gh)-1,4-benzodiazonin-3-one;
le 20-méthylcarbamate de téléocidine;
le 20-n-butylcarbamate de téléocidine;
le 20-méthylcarbamate de lyngbyatoxine A;
le 24-n-butylcarbamate de la lyngbyatoxine A;
le 20-éther méthylique 30-méthylcarbamate d'aplysiatoxine;
le 20-acétate 30-méthylcarbamate d'aplysiatoxine;
le 20,30-bis(méthylcarbamate) d'aplysiatoxine;
le 26-méthylcarbamate de bryostatine 1;
le 26-n-butylcarbamate de bryostatine 1.

**6.** Composé selon l'une quelconque des revendications 1 à 5, dans lequel la thérapie comprend le traitement de l'un quelconque des troubles ci-après: des conditions inflammatoires, le cancer, la leucémie, l'asthme, l'hypertension, des infections parasites, le psoriasis, les ulcères, l'arthrite, les maladies auto-immunes, les maladies amoébiques, la réplication virale HTLV-III/LAV et la maladie d'Alzheimer.

**7.** Composé selon l'une quelconque des revendications 1 à 5, dans lequel la thérapie est de type anti-nociceptif ou comprend une quelconque des thérapies associées à la sécrétion d'insuline ou à l'activité insulinomimétique, à la stimulation de la production des lymphokines (par exemple l'interféron) ou des interleukines, à la production du facteur nécrosant des tumeurs, à la stimulation et/ou la reconstitution du système immun, à l'accélération de la guérison de blessures et à l'amélioration de fonctions du système nerveux central, par exemple la mémoire et l'apprentissage.

**8.** Antagoniste de la protéine kinase C, agoniste non toxique de la protéine kinase C, antagoniste des phorboïdes toxiques ou encore agoniste non toxique de type phorboïde comprenant un composé tel que défini dans l'une quelconque des revendications 1 à 5, à l'exclusion:

a) des composés diterpènes contenant un groupe carboxyle, un groupe carboxaldéhyde, un groupe carboxaldéhyde-2,4-dinitrophényl-hydrazone, un groupe cyano, un groupe alcanoyloxyméthylène, un groupe chlorométhyle ou méthyle d'alcoxyméthylène à la place du groupe hydroxyméthyle;
b) des composés d'indole contenant un groupe chlorométhyle, un groupe méthyle, un groupe alcanoyloxyméthylène ou un groupe alcoxyméthylène à la place du groupe hydroxyméthyle.

**9.** Utilisation, pour la préparation d'un médicament destiné au traitement de conditions dans lesquelles est impliquée la protéine kinase C de manière pathologique ou thérapeutique, d'un composé non toxique dérivé d'un composé phorboïde mère toxique qui se lie de manière réversible ou irréversible à un récepteur du type du diacylglycérol et/ou qui active n'importe quelle forme de la protéine-kinase C et qui contient un groupe hydroxyméthyle ou un groupe 1-hydroxyéthyle lié à un atome de carbone, dans lequel (a) le groupe hydroxyméthyle ou le groupe 1-hydroxyéthyle a été remplacé par un groupe substituant lié de manière simple ou double audit atome de carbone, le remplacement donnant lieu à la perte des propriétés toxiques; ou (b) le groupe hydroxyméthyle ou le groupe 1-hydroxyéthyle est absent et un groupe substituant qui donne lieu à la perte des propriétés toxiques est lié de manière simple ou double à un atome de carbone immédiatement adjacent à l'atome de carbone auquel est lié le groupe hydroxyméthyle ou le groupe 1-hydroxyéthyle dans le composé mère.

**10.** Utilisation d'un composé tel que défini dans l'une quelconque des revendications 1 à 5 ou encore dans la revendication 9, pour la préparation d'un médicament pour le traitement de l'un quelconque des troubles ci-après: des conditions inflammatoires, le cancer, la leucémie, l'asthme, l'hypertension, des infections parasites, le psoriasis, les ulcères, l'arthrite, les maladies auto-immunes, les maladies amoébiques, la réplication virale HTLV-III/LAV et la maladie d'Alzheimer.

**11.** Utilisation d'un composé tel que défini dans l'une quelconque des revendications 1 à 5 ou encore à la revendication 9, pour la préparation d'un médicament à utiliser dans la thérapie du type anti-nociceptif ou comprend une quelconque des thérapies associées à la sécrétion d'insuline, à l'activité insulinomimétique ou encore à la stimulation de la production des lymphokines (par exemple l'interféron) ou des interleukines, à la production du facteur nécrosant des tumeurs, à la stimulation et/ou la reconstitution du système immun, à l'accélération de la guérison de blessures et à l'amélioration de fonctions du système nerveux central, par exemple la mémoire et l'apprentissage.

**12.** Composition pharmaceutique contenant un composé tel que défini dans l'une quelconque des revendications 1 à 5 ou encore à la revendication 9, ainsi qu'un support pharmaceutiquement acceptable.

**13.** Procédé de préparation d'une substance à utiliser en thérapie, comprenant un antagoniste non toxique des phorboïdes ou encore un ligand agoniste non inflammatoire sélectif pour un ou plusieurs récepteurs des phorboïdes ou un ou plusieurs sous-types de la protéine kinase C, comprenant le fait de remplacer un groupe hydroxyméthyle ou un groupe 1-hydroxyéthyle lié à un atome de carbone dans un composé phorboïde mère toxique, ledit composé phorboïde mère se liant de manière réversible ou irréversible à un récepteur du type du diacylglycérol et/ou activant n'importe quelle forme de la protéine kinase C, par un groupe lié de manière simple ou double à un atome de carbone auquel est lié le groupe hydroxyméthyle ou le groupe 1-hydroxyéthyle dans le composé phorboïde toxique ou encore à un atome de carbone immédiatement adjacent, et qui est représenté par $S_0$-$E_0$ où $S_0$ représente une chaîne d'atomes saturée ou insaturée, droite ou ramifiée, qui sépare $E_0$ du reste du composé d'un nombre linéaire d'au moins 2 atomes, mais qui n'est pas supérieur à 6 atomes, et contient:

de 1 à 6 hétéroatomes choisis parmi un atome d'oxygène, un atome d'azote et un atome de soufre;
un atome de phosphore; ou
jusqu'à 12 atomes d'halogène;
avec cette réserve que le nombre total d'atomes ne dépasse pas 35; et
$E_0$ comprend un groupe saturé ou à insaturation simple ou multiple contenant jusqu'à 8 atomes de carbone et, le cas échéant, de 1 à 12 atomes d'halogène, de 1 à 4 hétéroatomes choisis, indépendamment l'un de l'autre, parmi un atome d'oxygène, un atome d'azote, un atome de soufre; ainsi qu'un atome de phosphore; ou dans lequel
$S_0$-$E_0$ pris ensemble peuvent représenter un atome d'hydrogène, un atome d'halogène ou un atome d'oxygène cétonique ou encore un groupe hydroxyle, un groupe amine ou un groupe thiol lié de manière simple ou double à l'atome de carbone, en remplacement du groupe hydroxyméthyle ou du groupe 1-hydroxyéthyle.

**14.** Utilisation d'un composé tel que défini dans l'une quelconque des revendications 1 à 5 ou encore à la revendication 9, dans le diagnostic in vitro, par exemple dans un dosage pour détecter la teneur en protéine kinase C.

**15.** Utilisation in vitro d'un composé tel que défini dans l'une quelconque des revendications 1 à 5 ou à la revendication 9, à titre de ligand sélectif d'un sous-type de récepteur des phorboïdes et/ou d'un sous-type de la protéine kinase C.

**16.** Utilisation d'un composé tel que défini dans l'une quelconque des revendications 1 à 5 ou à la revendication 9, comme outil pharmacologique pour la recherche in vitro.

**17.** Utilisation d'un composé tel que défini dans l'une quelconque des revendications 1 à 5 ou à la revendication 9 (à l'exclusion de la résinifératoxine et de la tinyatoxine), comme outil pharmacologique pour la recherche in vivo en excluant des procédés de traitement du corps humain ou animal par des procédés de thérapie et de diagnostic pratiqués sur le corps humain ou sur le corps d'un animal.

**18.** Utilisation d'un composé tel que défini dans l'une quelconque des revendications 1 à 5 ou à la revendication 9, comme produit intermédiaire dans la préparation d'un autre médicament.

**19.** Composé répondant à la formule:

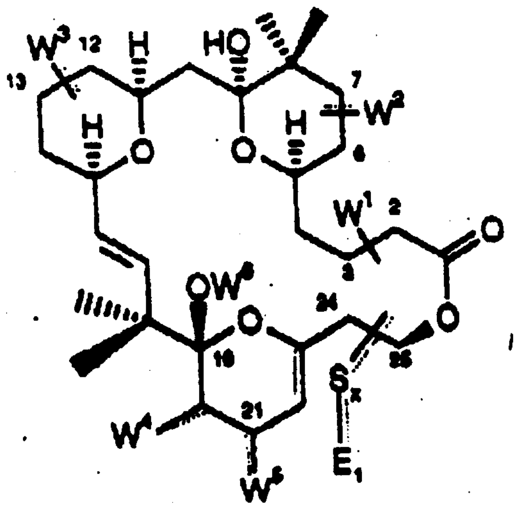

I

sous la forme d'un isomère individuel, d'un mélange d'isomères, d'un produit racémique, d'un antipode optique ou encore un de ses sels pharmaceutiquement acceptables,
dans laquelle $W^1$ et $W^2$ sont choisis parmi un atome d'hydrogène, un atome d'halogène, un groupe hydroxyle, un groupe alcoxy en $C_1$-$C_5$, un groupe alcanoyl(en $C_1$-$C_5$)oxy et un groupe cyano; $W^3$-$W^5$ peuvent représenter chacun un atome d'hydrogène ou encore un groupe à chaîne droite ou ramifiée, cyclique ou acyclique, saturé, insaturé contenant des hétéroatomes et/ou des atomes de carbone aromatiques, qui ne contient pas plus de 30 atomes de carbone, pas plus de 24 atomes d'halogène et pas plus de 8 hétéroatomes choisis parmi un atome d'oxygène, un atome d'azote et un atome de soufre, et $W^4$ et $W^5$ pris ensemble peuvent former un noyau carbocyclique ou hétérocyclique supplémentaire; et $W^6$ représente un atome d'hydrogène;
dans laquelle $S_xE_1$ comprend un groupe substituant lié à l'atome de carbone 25, où $S_x$ représente une chaîne d'atomes saturée ou insaturée, droite ou ramifiée, qui sépare $P_6$ et $E_1$ d'un nombre linéaire maximal de 6 atomes et qui peut contenir de 1 à 6 hétéroatomes choisis parmi un atome d'oxygène, un atome d'azote et un atome de soufre; un atome de phosphore; ou un nombre d'atomes d'halogène allant jusque 12; avec cette réserve que le nombre total des atomes ne dépasse pas 35; et
dans laquelle $E_1$ comprend un groupe saturé ou à insaturation simple ou multiple contenant jusqu'à 8 atomes de carbone et contenant, le cas échéant, de 1 à 12 atomes d'halogène, de 1 à 4 hétéroatomes choisis, indépendamment l'un de l'autre, parmi un atome d'oxygène, un atome d'azote et un atome de soufre; ainsi qu'un atome de phosphore, ou encore dans laquelle
$S_xE_1$ pris ensemble peuvent représenter un atome d'hydrogène, un atome d'halogène ou un atome d'oxygène cétonique ou encore un groupe hydroxyle, un groupe amine ou un groupe thiol lié de manière simple ou double à un atome de carbone d'un composé mère correspondant pour remplacer un groupe hydroxyméthyle ou un groupe 1-hydroxyéthyle dudit composé mère, avec cette réserve que $S_xE_1$ ne représente pas un groupe hydroxyméthyle, un groupe 1-hydroxyéthyle ou encore un groupe 1-hydroxyéthyle acylé.